# EUROPEAN PATENT SPECIFICATION

(11) **EP 2 521 715 B1**
(45) Date of publication and mention of the grant of the patent: **25.06.2014**
(21) Application number: 11702702.9
(22) Date of filing: 04.01.2011
(51) Int. Cl.: C07D 213/56, C07D 213/64, C07D 295/185, A61K 31/495, A61K 31/496, A61P 33/02, A61P 33/06

(54) **PIPERAZINES AS ANTIMALARIAL AGENTS**
PIPERAZINE ALS ANTIMALARIAMITTEL
PIPÉRAZINES EN TANT QU'AGENTS ANTIPALUDIQUES

(30) Priority: 10.05.2010 WO PCT/IB2010/052045; 05.01.2010 WO PCT/IB2010/050022
(43) Date of publication of application: 14.11.2012
(73) Proprietor: Actelion Pharmaceuticals Ltd., 4123 Allschwil (CH)
(72) Inventor: AISSAOUI, Hamed, CH-4123 Allschwil (CH); BOSS, Christoph, CH-4123 Allschwil (CH); CORMINBOEUF, Olivier, CH-4123 Allschwil (CH); HEIDMANN, Bibia, CH-4123 Allschwil (CH); SIEGRIST, Romain, CH-4123 Allschwil (CH)
(74) Representative: Gschwend, Thomas Peter
(86) International application number: PCT/IB2011/050009
(87) International publication number: WO 2011/083413

(56) References cited:
- WO-A1-2007/046075

## Description

The invention relates to novel compounds of the formula I. The invention also concerns related aspects including processes for the preparation of the compounds, pharmaceutical compositions containing one or more compounds of the formula I and especially their use as medicaments to treat or prevent malaria infections or to treat or prevent other protozoal diseases like sleeping sickness, Chagas disease, amebiasis, giardiasis, trichomoniasis, toxoplasmosis, and leishmaniasis.

### Background of the invention:

Numerous serious diseases affecting humans as well as domestic and livestock animal are caused by protozoal organisms such as *kinetoplastida, apicomplexa, anaerobic protozoa, microsporidia* and *plasmodium,* for example. The clinically most relevant of these diseases is malaria.

Malaria is one of the most serious and complex health problems affecting humanity in the 21^{st} century. The disease affects about 300 million people worldwide, killing 1 to 1.5 million people every year. Malaria is an infectious disease caused by four species of the protozoan parasite *plasmodium, P. falciparum* being the most severe of the four. All attempts to develop vaccines against *P. falciparum* have failed so far. Therefore, therapies and preventive measures against malaria are confined to drugs. Various classes of antimalarial drugs exist. The most widely used are the quinoline antimalarials, e.g. chloroquine which has been an especially effective drug for both prophylaxis and therapy. However, resistance to many of the currently available antimalarial drugs is spreading rapidly, threatening people in areas where malaria is endemic. Reports of multi-drug resistant strains of malaria parasites render the search for new antimalarial agents especially urgent. *P. falciparum* enters the human body by way of bites of the female anophelino mosquito (it may also be transmitted by blood transfusion from asymptotic donors; almost all infected blood components including red cells, platelet concentrates, white cells, cryoprecipitates and fresh plasma can transmit malaria). The *plasmodium* parasite initially populates the liver, and during later stages of the infectious cycle reproduces in red blood cells. During this stage, the parasite degrades hemoglobin and uses the degradation products as nutrients for growth.

The limitations of the current antiprotozoal chemotherapeutic arsenal underscore the need for new drugs in this therapeutic area. The present invention relates to the identification of novel low molecular weight, non-peptidic, non-quinoline compounds of formula I which are useful in the treatment and/or prevention of protozoal infections, especially in the treatment and/or prevention of malaria, in particular plasmodium falciparum malaria.

WO 2007/046075 also discloses piperazine derivatives as antimalarial agents. The compound of WO 2007/046075 which comes closest to some of the presently claimed compounds is the compound of Example 54 which corresponds to reference Example 1 herein. However, the presently claimed compounds which come structurally closest to the compound of Example 54 of WO 2007/046075 exhibit an *in vitro* activity against erythrocytic stages of the P. falciparum strain NF54 in the presence of 50% serum which is significantly higher compared to the compound of Example 54 of WO 2007/046075 (see Table 1 below).

### Detailed description of the invention:

i) The present invention relates to novel compounds of the formula I: 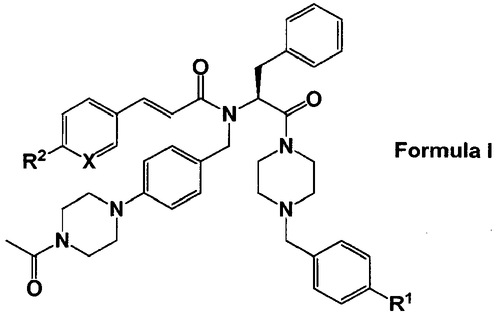 wherein
   ◆ X is CH or N;
      R¹ represents -NO₂, -N(CH₃)₂, or -NCH₃(CH₂CH₂OH); and
      R² represents hydrogen, methyl, ethyl, n-propyl, isopropyl, tert-butyl, cyano, halogen, methoxy, ethoxy, n-propoxy, isopropoxy, trifluoromethyl, difluoromethoxy, methylsulfonyl, acetyl, or acetylamino; or
   ◆ X is CH, **R¹** is hydrogen, and **R²** is ethyl, isopropyl, tert-butyl, ethoxy, n-propoxy, isopropoxy, methylsulfonyl, acetylamino, or methoxycarbonyl; or
   ◆ **X** is CH, **R¹** is cyano, and **R²** is ethyl, isopropyl, tert-butyl, ethoxy, n-propoxy, isopropoxy, trifluoromethyl, difluoromethoxy, trifluoromethoxy, methylsulfonyl, or acetylamino; or
   ◆ **X** is CH, **R¹** is chloro, and **R²** is ethyl, isopropyl, tert-butyl, ethoxy, n-propoxy, isopropoxy, difluoromethoxy, methylsulfonyl, or acetylamino; or
   ◆ **X** is CH, **R¹** is methoxy or isopropoxy, and **R²** is trifluoromethyl; or
   ◆ **X** is CH, **R¹** is methylsulfonyl or ethylsulfonyl, and **R²** is trifluoromethyl, ethyl, isopropyl, tert-butyl, ethoxy, n-propoxy, isopropoxy, or difluoromethoxy, such as especially trifluoromethyl, tert-butyl, n-propoxy, or isopropoxy.
ii) A further embodiment of the invention relates to compounds of the formula I according to embodiment i), wherein
   ◆ **X** is CH or N;
      **R¹** represents -NO₂, -N(CH₃)₂, or -NCH₃(CH₂CH₂OH); and
      **R²** represents hydrogen, methyl, ethyl, n-propyl, isopropyl, tert-butyl, cyano, halogen, methoxy, ethoxy, n-propoxy, isopropoxy, trifluoromethyl, difluoromethoxy, methylsulfonyl, acetyl, or acetylamino; or
   ◆ **X** is CH, **R¹** is hydrogen, and **R²** is ethyl, isopropyl, tert-butyl, ethoxy, n-propoxy, isopropoxy, methylsulfonyl, acetylamino, or methoxycarbonyl; or
   ◆ **X** is CH, **R¹** is cyano, and **R²** is ethyl, isopropyl, tert-butyl, ethoxy, n-propoxy, isopropoxy, trifluoromethyl, difluoromethoxy, trifluoromethoxy, methylsulfonyl, or acetylamino; or
   ◆ **X** is CH, **R¹** is chloro, and **R²** is ethyl, isopropyl, tert-butyl, ethoxy, n-propoxy, isopropoxy, difluoromethoxy, methylsulfonyl, or acetylamino; or
   ◆ **X** is CH, **R¹** is methoxy or isopropoxy, and **R²** is trifluoromethyl.
iii) A further embodiment of the invention relates to compounds of the formula I according to embodiment i), wherein
   ◆ **X** is CH or N;
      **R¹** represents -NO₂, -N(CH₃)₂, or -NCH₃(CH₂CH₂OH); and
      **R²** represents ethyl, isopropyl, tert-butyl, methoxy, ethoxy, n-propoxy, isopropoxy, trifluoromethyl, difluoromethoxy, methylsulfonyl, or acetylamino; or
   ◆ **X** is CH, **R¹** is hydrogen, and **R²** is ethyl, isopropyl, tert-butyl, ethoxy, n-propoxy, isopropoxy, methylsulfonyl, acetylamino, or methoxycarbonyl; or
   ◆ **X** is CH, **R¹** is cyano, and **R²** is ethyl, isopropyl, tert-butyl, ethoxy, n-propoxy, isopropoxy, trifluoromethyl, difluoromethoxy, trifluoromethoxy, methylsulfonyl, or acetylamino; or
   ◆ **X** is CH, **R¹** is chloro, and **R²** is ethyl, isopropyl, tert-butyl, ethoxy, n-propoxy, isopropoxy, difluoromethoxy, methylsulfonyl, or acetylamino; or
   ◆ **X** is CH, **R¹** is methoxy or isopropoxy, and **R²** is trifluoromethyl.
iv) A further embodiment of the invention relates to compounds of the formula I according to embodiment i), wherein
   **R¹** represents -NO₂, -N(CH₃)₂, or -NCH₃(CH₂CH₂OH); and
   **R²** represents hydrogen, methyl, ethyl, n-propyl, isopropyl, tert-butyl, cyano, halogen, methoxy, ethoxy, n-propoxy, isopropoxy, trifluoromethyl, difluoromethoxy, methylsulfonyl, acetyl, or acetylamino.
v) A further embodiment of the invention relates to compounds of the formula I according to embodiment i), wherein
   **R¹** represents -NO₂, -N(CH₃)₂, or -NCH₃(CH₂CH₂OH); and
   **R²** represents ethyl, isopropyl, tert-butyl, methoxy, ethoxy, n-propoxy, isopropoxy, trifluoromethyl, difluoromethoxy, methylsulfonyl, or acetylamino.
vi) A further embodiment of the invention relates to compounds of the formula I according to embodiment iv) or v), wherein **X** is CH.
vii) A further embodiment of the invention relates to compounds of the formula I according to embodiment iv) or v), wherein X is N.
viii) A further embodiment of the invention relates to compounds of the formula I according to any one of embodiments iv) to vii), wherein **R¹** represents -NO₂.
ix) A further embodiment of the invention relates to compounds of the formula I according to any one of embodiments iv) to vii), wherein **R¹** represents -N(CH₃)₂.
x) A further embodiment of the invention relates to compounds of the formula I according to any one of embodiments iv) to vii), wherein **R¹** represents -NCH₃(CH₂CH₂OH).
xi) A further embodiment of the invention relates to compounds of the formula I according to any one of embodiments iv) to x), wherein **R²** is ethyl, isopropyl, tert-butyl, ethoxy, n-propoxy, or isopropoxy.
xii) A further embodiment of the invention relates to compounds of the formula I according to embodiment xi), wherein **R²** is isopropoxy.
xiii) A further embodiment of the invention relates to compounds of the formula I according to any one of embodiments iv) to x), wherein **R²** is trifluoromethyl, difluoromethoxy, methylsulfonyl, or acetylamino.
xiv) A further embodiment of the invention relates to compounds of the formula I according to any one of embodiments iv) to x), wherein **R²** is methoxy.
xv) A further embodiment of the invention relates to compounds of the formula I according to any one of embodiments iv) to x), wherein **R²** is hydrogen, methyl, n-propyl, cyano, halogen, or acetyl.

The term "halogen" as used herein means fluorine, chlorine, bromine or iodine, such as especially fluorine or chlorine.

Where the plural form is used for compounds, salts, pharmaceutical compositions, diseases and the like, this is intended to mean also a single compound, salt, or the like.

Any reference hereinbefore or hereinafter to a compound of formula I is to be understood as referring also to salts, especially pharmaceutically acceptable salts, of a compound of formula I, as appropriate and expedient.

The term "pharmaceutically acceptable salts" refers to non-toxic, inorganic or organic acid and/or base addition salts. Reference can be made to "Salt selection for basic drugs", Int. J. Pharm. 1986, 33, 201-17.

The present invention also includes isotopically labelled, especially ²H (deuterium) labelled compounds of formula I, which compounds are identical to the compounds of formula I except that one or more atoms have each been replaced by an atom having the same atomic number but an atomic mass different from the atomic mass usually found in nature. Isotopically labelled, especially ²H (deuterium) labelled compounds of formula I and salts thereof are within the scope of the present invention. Substitution of hydrogen with the heavier isotope ²H (deuterium) may lead to greater metabolic stability, resulting e.g. in increased *in vivo* half-life or reduced dosage requirements, or may lead to reduced inhibition of cytochrome P450 enzymes, resulting e.g. in an improved safety profile. In one embodiment of the invention, the compounds of formula I are not isotopically labelled, or they are labelled only with one or more deuterium atoms. In a sub-embodiment, the compounds of formula I are not isotopically labelled at all. Isotopically labelled compounds of formula I may be prepared in analogy to the methods described hereinafter, but using the appropriate isotopic variation of suitable reagents or starting materials.

Examples of preferred compounds of formula I are selected from the group consisting of:
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-trifluoromethyl-phenyl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(6-trifluoromethyl-pyridin-3-yl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(6-methoxy-pyridin-3-yl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(6-ethoxy-pyridin-3-yl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-ethoxy-phenyl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-methanesulfonyl-phenyl)-acrylamide,
(S)-3-(4-Acetylamino-phenyl)-N-[4-(4-acetyl-piperazin-1-yl)-benzyl]-N-{1-benzyl-2-[4-(4-dimethylamino-benzyl)-piperazin-1-yl]-2-oxo-ethyl}-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-propoxy-phenyl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-isopropoxy-phenyl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-ethyl-phenyl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-difluoromethoxy-phenyl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-tert-butyl-phenyl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-isopropyl-phenyl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-trifluoromethyl-phenyl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(6-trifluoromethyl-pyridin-3-yl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(6-methoxy-pyridin-3-yl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(6-ethoxy-pyridin-3-yl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-ethoxy-phenyl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-methanesulfonyl-phenyl)-acrylamide,
(S)-3-(4-Acetylamino-phenyl)-N-[4-(4-acetyl-piperazin-1-yl-benzyl]-N-[1-benzyl-2-(4-{4-[(2-hydroxy-ethyl)-methyl-amino]-benzyl}-piperazin-1-yl)-2-oxo-ethyl]-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-propoxy-phenyl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-isopropoxy-phenyl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-ethyl-phenyl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-difluoromethoxy-phenyl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-isopropyl-phenyl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-tert-butyl-phenyl)-acrylamide, and
(S)-N-[4-(4-Acetyl-piperazin-1-yl)-benzyl]-N-{1-benzyl-2-[4-(4-nitro-benzyl)-piperazin-1-yl]-2-oxo-ethyl}-3-(4-trifluoromethyl-phenyl)-acrylamide.

Further examples of preferred compounds of formula I are selected from the group consisting of:
(S)-N-[4-(4-Acetyl-piperazin-1-yl)benzyl]-N-{1-(4-(4-(dimethylamino)benzyl)piperazin-1-yl)-1-oxo-3-phenylpropan-2-yl}cinnamamide,
(S)-N-[4-(4-Acetyl-piperazin-1-yl)benzyl]-N-{1-[4-(4-(dimethylamino)benzyl)piperazin-1-yl]-1-oxo-3-phenylpropan-2-yl}-3-(p-tolyl)acrylamide,
(S)-N-[4-(4-Acetyl-piperazin-1-yl)benzyl]-N-{1-(4-(4-(dimethylamino)benzyl)piperazin-1-yl)-1-oxo-3-phenylpropan-2-yl}-3-(4-propylphenyl)acrylamide,
()-N-[4-(4-Acetyl-piperazin-1-yl)benzyl]-N-{1-(4-(4-(dimethylamino)benzyl)piperazin-1-yl)-1-oxo-3-phenylpropan-2-yl}-3-(4-methoxyphenyl)acrylamide,
(S)-3-(4-Acetyl-phenyl)-N-[4-(4-acetyl-piperazin-1-yl)benzyl]-N-{1-(4-(4-(dimethylamino)benzyl)piperazin-1-yl)-1-oxo-3-phenylpropan-2-yl}acrylamide,
(S)-N-[4-(4-Acetyl-piperazin-1-yl)benzyl]-3-(4-cyanophenyl)-N-{1-(4-(4-(dimethylamino)benzyl)piperazin-1-yl)-1-oxo-3-phenylpropan-2-yl}acrylamide,
(S)-N-[4-(4-Acetyl-piperazin-1-yl)benzyl]-N-{1-(4-(4-(dimethylamino)benzyl)piperazin-1-yl)-1-oxo-3-phenylpropan-2-yl}-3-(4-fluorophenyl)acrylamide, and
(S)-N-[4-(4-Acetyl-piperazin-1-yl)benzyl]-3-(4-chlorophenyl)-N-{1-(4-(4-(dimethylamino)benzyl)piperazin-1-yl)-1-oxo-3-phenylpropan-2-yl}acrylamide.

Further examples of preferred compounds of formula I are selected from the group consisting of:
(S)-N-[4-(4-Acetyl-piperazin-1-yl)-benzyl]-N-[1-benzyl-2-(4-benzyl-piperazin-1-yl)-2-oxo-ethyl]-3-(4-ethyl-phenyl)-acrylamide,
(S)-N-[4-(4-Acetyl-piperazin-1-yl)-benzyl]-N-[1-benzyl-2-(4-benzyl-piperazin-1-yl)-2-oxo-ethyl]-3-(4-tert-butyl-phenyl)-acrylamide,
(S)-N-[4-(4-Acetyl-piperazin-1-yl)-benzyl]-N-[1-benzyl-2-(4-benzyl-piperazin-1-yl)-2-oxoethyl]-3-(4-ethoxy-phenyl)-acrylamide,
(S)-4-(2-{[4-(4-Acetyl-piperazin-1-yl)-benzyl]-[1-benzyl-2-(4-benzyl-piperazin-1-yl)-2-oxoethyl]-carbamoyl}-vinyl)-benzoic acid methyl ester,
(S)-N-[4-(4-Acetyl-piperazin-1-yl)-benzyl]-N-[1-benzyl-2-(4-benzyl-piperazin-1-yl)-2-oxo-ethyl]-3-(4-methanesulfonyl-phenyl)-acrylamide,
(S)-3-(4-Acetylamino-phenyl)-N-[4-(4-acetyl-piperazin-1-yl)-benzyl]-N-[1-benzyl-2-(4-benzyl-piperazin-1-yl)-2-oxo-ethyl]-acrylamide,
(S)-N-[4-(4-Acetyl-piperazin-1-yl)-benzyl]-N-[1-benzyl-2-(4-benzyl-piperazin-1-yl)-2-oxoethyl]-3-(4-propoxy-phenyl)-acrylamide,
(S)-N-[4-(4-Acetyl-piperazin-1-yl)-benzyl]-N-[1-benzyl-2-(4-benzyl-piperazin-1-yl)-2-oxoethyl]-3-(4-isopropoxy-phenyl)-acrylamide,
(S)-N-[4-(4-Acetyl-piperazin-1-yl)-benzyl]-N-[1-benzyl-2-(4-benzyl-piperazin-1-yl)-2-oxo-ethyl]-3-(4-isopropyl-phenyl)-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-trifluoromethyl-phenyl)-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-ethoxy-phenyl)-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-methanesulfonyl-phenyl)-acrylamide,
(S)-3-(4-Acetylamino-phenyl)-N-[4-(4-acetyl-piperazin-1-yl)-benzyl]-N-{1-benzyl-2-[4-(4-cyano-benzyl)-piperazin-1-yl]-2-oxo-ethyl}-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-propoxy-phenyl)-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-isopropoxy-phenyl)-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-ethyl-phenyl)-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-difluoromethoxy-phenyl)-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-isopropyl-phenyl)-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-trifluoromethoxy-phenyl)-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-tert-butyl-phenyl)-acrylamide,
(S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-ethoxy-phenyl)-acrylamide,
(S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-methanesulfonyl-phenyl)-acrylamide,
(S)-3-(4-Acetylamino-phenyl)-N-[4-(4-acetyl-piperazin-1-yl)-benzyl]-N-{1-benzyl-2-[4-(4-chloro-benzyl)-piperazin-1-yl]-2-oxo-ethyl}-acrylamide,
(S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-propoxy-phenyl)-acrylamide,
(S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-isopropoxy-phenyl)-acrylamide,
(S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-ethyl-phenyl)-acrylamide,
(S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-difluoromethoxy-phenyl)-acrylamide,
(S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-isopropyl-phenyl)-acrylamide,
(S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-tert-butyl-phenyl)-acrylamide,
(S)-N-[4-(4-Acetyl-piperazin-1-yl)-benzyl]-N-{1-benzyl-2-[4-(4-methoxy-benzyl)-piperazin-1-yl]-2-oxo-ethyl}-3-(4-trifluoromethyl-phenyl)-acrylamide, and
(S)-N-[4-(4-Acetyl-piperazin-1-yl)-benzyl]-N-{1-benzyl-2-[4-(4-isopropoxy-benzyl)-piperazin-1-yl]-2-oxo-ethyl}-3-(4-trifluoromethyl-phenyl)-acrylamide.

Further examples of preferred compounds of formula I are selected from the group consisting of:
(S)-N-[4-(4-Acetyl-piperazin-1-yl)-benzyl]-N-{1-benzyl-2-[4-(4-(methylsulfonyl)-benzyl)-piperazin-1-yl]-2-oxo-ethyl}-3-(4-isopropoxyphenyl)-acrylamide,
(S)-N-[4-(4-Acetyl-piperazin-1-yl)-benzyl]-N-{1-benzyl-2-[4-(4-(methylsulfonyl)-benzyl)-piperazin-1-yl]-2-oxo-ethyl}-3-(4-tert-butyl-phenyl)-acrylamide,
(S)-N-[4-(4-Acetyl-piperazin-1-yl)-benzyl]-N-{1-benzyl-2-[4-(4-(methylsulfonyl)-benzyl)-piperazin-1-yl]-2-oxo-ethyl}-3-(4-propoxyphenyl)-acrylamide,
(S)-N-(4-(4-acetylpiperazin-1-yl)benzyl)-N-(1-(4-(4-(methylsulfonyl)benzyl)piperazin-1-yl)-1-oxo-3-phenylpropan-2-yl)-3-(4-(trifluoromethyl)phenyl)acrylamide,
(S)-N-[4-(4-Acetyl-piperazin-1-yl)-benzyl]-N-{1-benzyl-2-[4-(4-(ethylsulfonyl)-benzyl)-piperazin-1-yl]-2-oxo-ethyl}-3-(4-propoxyphenyl)-acrylamide, and
(S)-N-(4-(4-acetylpiperazin-1-yl)benzyl)-N-(1-(4-(4-(ethylsulfonyl)benzyl)piperazin-1-yl)-1-oxo-3-phenylpropan-2-yl)-3-(4-(trifluoromethyl)phenyl)acrylamide.

The compounds of formula I and their pharmaceutically acceptable salts can be used as medicaments, e.g. in the form of pharmaceutical compositions for enteral or parenteral administration, and are suitable for the treatment and/or prevention of the diseases mentioned herein, such as especially malaria.

The production of the pharmaceutical compositions can be effected in a manner which will be familiar to any person skilled in the art (see for example Remington, The Science and Practice of Pharmacy, 21st Edition (2005), Part 5, "Pharmaceutical Manufacturing" [published by Lippincott Williams & Wilkins]) by bringing the described compounds of formula I or their pharmaceutically acceptable salts, optionally in combination with other therapeutically valuable substances, into a galenical administration form together with suitable, non-toxic, inert, pharmaceutically acceptable solid or liquid carrier materials and, if desired, usual pharmaceutical adjuvants.

The compounds of formula I can be used in a method for the treatment or prevention of the diseases mentioned herein, such as especially malaria, said method comprising administering to a subject a pharmaceutically active amount of a compound of formula I.

The compounds of formula I or the above-mentioned pharmaceutical compositions may also be used in combination with one or more other therapeutically useful substances e.g. with other antimalarials like quinolines (e.g. quinine, chloroquine, amodiaquine, mefloquine, primaquine, and tafenoquine), peroxide antimalarials (e.g. artemisinin, artemether, and artesunate), pyrimethamine-sulfadoxine antimalarials (e.g. Fansidar®), hydroxynaphtoquinones (e.g. atovaquone), acroline-type antimalarials (e.g. pyronaridine), and other antiprotozoal agents like ethylstibamine, hydroxystilbamidine, pentamidine, stilbamidine, quinapyramine, puromycine, propamidine, nifurtimox, melarsoprol, nimorazole, nifuroxime, aminitrozole and the like.

The present invention also relates to the use of a compound of formula I for the preparation of a pharmaceutical composition, optionally for use in combination with one or more other therapeutically useful substances such as those mentioned in the preceding paragraph, for the prevention and/or treatment of the diseases mentioned herein, such as especially malaria.

The compounds of the formula I of the present invention may be prepared according to the procedures described herein, especially as described in the experimental part.

In general, all chemical transformations can be performed according to well-known standard methodologies as described in the literature or as described in the procedures below.

Preparation of compounds of formula I, except for compounds wherein R¹ is -NCH₃(CH₂CH₂OH):

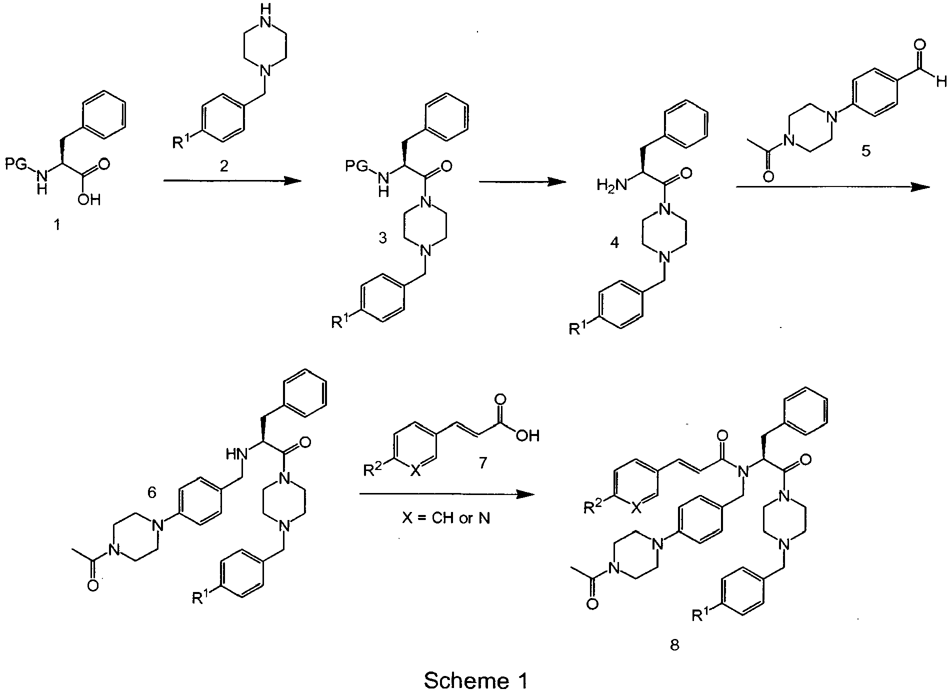

The Boc-Phe-OH 1 can be coupled with the benzylpiperazine derivative 2 via a peptidic coupling using activating agents such as TBTU (or PyBOP/HOBt) in the presence of a base such as DIPEA (or NEM) in DCM (or DMF) at RT to afford the intermediate 3. Alternatively, Cbz-Phe-OH can also be used in the initial peptidic coupling step to give 3. Boc-deprotection is usually achieved by reacting 3 with a solution of HCl 4N in dioxane using DCM as solvent, while Cbz-deprotection is achieved by hydrogenation with Pd/C catalyst in MeOH, to give the amine intermediate 4. Reductive amination between the free amine 4 and the aldehyde 5 at reflux in MeOH afforded the unstable imine (not depicted in the scheme), which is further reduced at RT with NaBH₄ to give the secondary amine intermediate 6. Alternatively, the reductive amination can be achieved in a solvent such as CH₃CN in the presence of a reducing reagent such as NaBH(OAc)₃ to give the expected secondary amine intermediate 6. Compound 6 can then be coupled with a carboxylic acid 7 using a peptidic coupling reagent such as TBTU, PyBOP/HOBt or the Ghosez's reagent in a solvent such as DCM (or DMF) at RT in the presence of a base such as DIPEA (or NEM). Alternatively, the carboxylic acid 7 can be converted to the corresponding acid chloride (not depicted in the scheme) using oxalyl chloride in DCM to give the final compounds 8 of formula **I.**

When R¹ = -NCH₃(CH₂CH₂OH) the compounds of formula I are prepared according to Scheme 2.

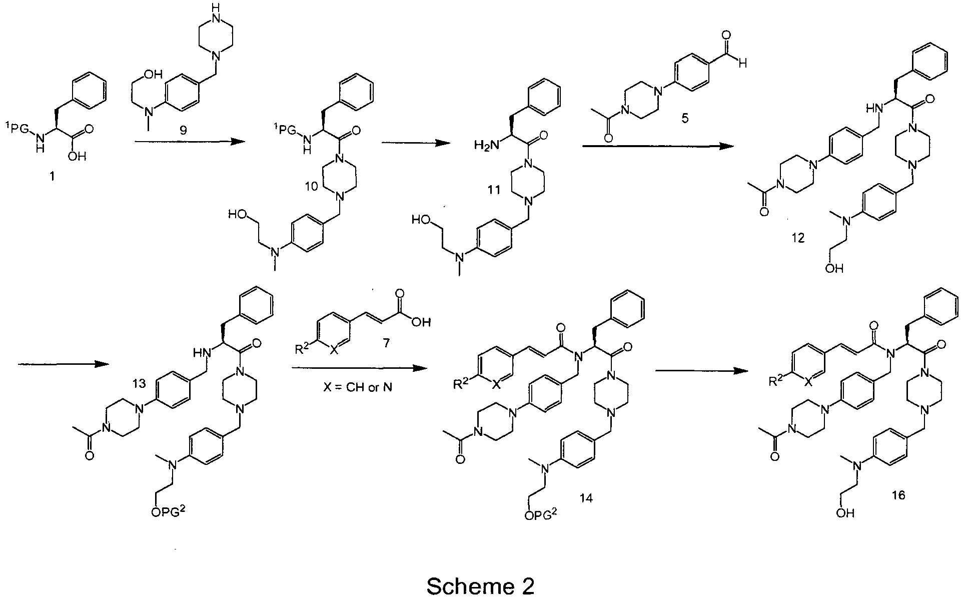

The Boc-Phe-OH 1 is coupled with the benzylpiperazine derivative 9 via a peptidic coupling reaction using activating agents such as TBTU (or PyBOP/HOBt) in the presence of a base such as DIPEA (or NEM) in DCM (or DMF) at RT to afford the intermediate 10. Alternatively, Cbz-Phe-OH can also be used in the initial peptide coupling step to give 10. Boc-deprotection is usually achieved by reacting **10** with a solution of HCl 4N in dioxane using DCM as solvent, while Cbz-deprotection is achieved by hydrogenation with Pd/C catalyst in MeOH, to give the amine intermediate 11. Reductive amination between the free amine 11 and the aldehyde 5 in CH₃CN at RT in the presence of a reducing agent such as NaBH(OAc)₃ affords the secondary amine intermediate **12.** The free hydroxyl group of compound **12** is protected using for instance TBDMSCI as silylating agent to give **13,** which is then coupled with a carboxylic acid **7** using a peptide coupling reagent such as TBTU (or PyBOP/HOBt) in a solvent such as DCM (or DMF) at RT in the presence of a base such as DIPEA. Alternatively, the carboxylic acid **7** can be activated by converting it to the corresponding acid chloride (not depicted in the scheme) using oxalyl chloride in DCM, to subsequently give compound **14.** Further deprotection under mild acidic conditions such as aqueous 1 M HCl in MeOH or fluorinated reagents such as TBAF yield the final compounds **16** of **formula I.**

The compounds of formula **I** can also be prepared according to the pathway depicted in Scheme 3.

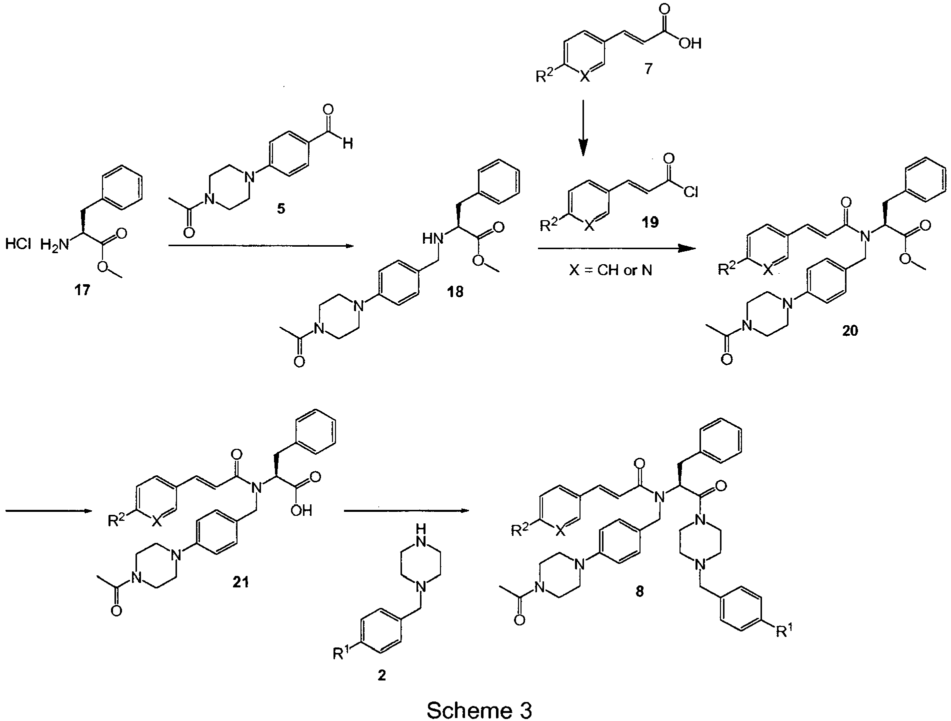

Reductive amination between the amino acid H-Phe-OMe.HCl 17 and the aldehyde 5 in MeOH under reflux affords the corresponding imine, which is further reduced to the secondary amine 18 in the presence of a reducing reagent such as NaBH₄ at RT. 18 can also be obtained using the conditions described above for compounds 6 and 12. The ester 18 is then coupled with the acid chloride 19 derived from the carboxylic acid 7 using oxalyl chloride in DCM or Ghosez's reagent. Alternatively, 18 can be directly coupled with the carboxylic acid 7 via a peptide coupling reaction using TBTU (or PyBOP/HOBt) as coupling agents in a solvent such as DCM (or DMF) at RT in the presence of a base such as DIPEA (or NEM). Careful saponification of the ester **20** with aqueous LiOH 0.5 N in THF at 0°C affords the acid **21.** Final peptide coupling with the benzylpiperazine **2** gives the final compounds **8** of **formula I.**

Benzylpiperazines **2** and **9** are commercially available and/or can be synthesized according to the following synthetic Scheme 4:

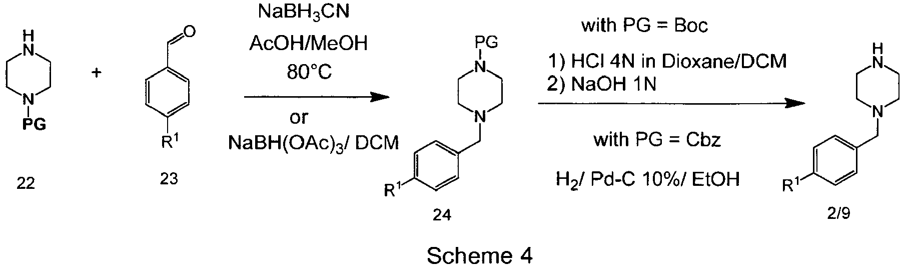

Cinnamic acids 7 are commercially available or/and can be synthesized according to the following pathways:

### Pathway A: Knoevenagel Reaction

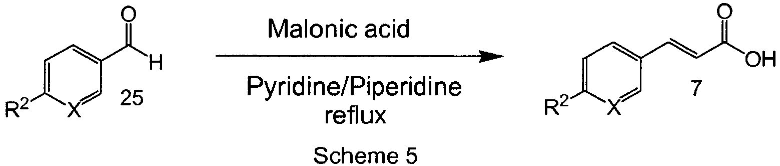

The cinnamic acids 7 are obtained by refluxing the aldehyde 25 with malonic acid in a mixture of piperidine/pyridine (WO 00/66566).

### Pathway B: Horner-Emmons Reaction

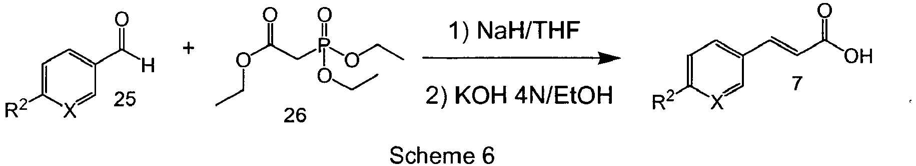

The cinnamic acids 7 are obtained in two steps by reacting the aldehydes 25 with triethyl phosphoacetate 26 in the presence of a base such as NaH in an aprotic solvent such as THF followed by saponification of the resulting ethyl ester with 4 N KOH in EtOH.

The following examples illustrate the present invention. All temperatures are stated in degrees Celsius and pressures in mbar. Unless mentioned otherwise, the reactions take place at RT. The ratio of amounts of solvents to one another is always stated in parts by volume. Chemical names for final products and intermediates have been generated on the basis of the chemical structural formulae with the aid of ChemDrawPro Automatic Nomenclature program.

### Analytic HPLC conditions:

(I) Agilent 1100 series with UV/Vis and MS detection (MS: Thermo Finnigan single quadrupole). Columns (4.6x50 mm, 5 µm): Waters X-Bridge C18 or Waters Atlantis T3. Basic conditions: eluents: A: MeCN, B: concentrated NH₃ in water (1.0 mL/L). Gradient 5 to 95% A over 1.5 min. Flow rate: 4.5 mL/min.
Acidic conditions: eluents A: water + 0.04% TFA, B: MeCN. Gradient 5 to 95% over 1.5 min. Flow rate 4.5 mL/min.

### Preparative HPLC conditions:

Gilson with UV/Vis + MS or UV/Vis + ELSD detection. Basic conditions: eluents: A: MeCN, B: H₂O + 0.5% NH₃ (25% aqueous).
(II) Waters X-Bridge column, 19x50 mm, 5 µm. Gradient: 20 to 90% A over 5 min. Flow rate: 40 mL/min.
(III) Waters X-Bridge column, 30x75 mm, 10 µm. Gradient: 20 to 90% A over 6 min. Flow rate: 75 mL/min.

**The following abbreviations are used herein:**
- AcOH: acetic acid
- Boc: tert.-butyloxycarbonyl
- Boc-Phe-OH: Boc-L-phenylanaline
- Cbz: benzyloxycarbonyl
- Cbz-Phe-OH: Cbz-L-phenylanaline
- DCM: dichloromethane
- DIPEA: N,N-diisopropylethylamine
- DMF: N,N-dimethylformamide
- Et: ethyl
- EtOH: ethanol
- EtOAc: ethyl acetate
- Et₂O: diethylether
- ELSD: evaporative light scattering detection
- h: hour(s)
- HOBt: hydroxybenzotriazole
- H-Phe-OMe.HCl: L-phenylalanine methylester hydrochloride
- HPLC: high performance liquid chromatography
- LC-MS: liquid chromatography - mass spectroscopy
- Me: methyl
- MeOH: methanol
- min: minute(s)
- MS: mass spectroscopy
- NaBH(OAc)₃: sodium triacetoxyborohydride
- NEM: N-ethyl morpholine
- PBS: phosphate buffered saline
- Pd/C: palladium on carbon
- PG: protecting group
- PyBOP: benzotriazol-1-yl-oxy-tris-pyrrolidinophosphonium hexafluorophosphate
- quant.: quantitative
- RT: room temperature
- Rt: retention time of a substance in HPLC (in minutes)
- TBAF: tetra-n-butylammonium fluoride
- TBDMSCI: tert-butyldimethyl chlorosilane
- TBTU: *O*-benzotriazol-1-yl-*N,N,N',N'*-tetramethyluronium tetrafluoroborate
- TFA: trifluoroacetic acid
- THF: tetrahydrofuran
- UV: ultra violet
- Vis: visible
- #: number

### Preparation of compounds of formula I via pathway depicted in Scheme 1 - General Procedures and Examples:

### General Method A - step 1

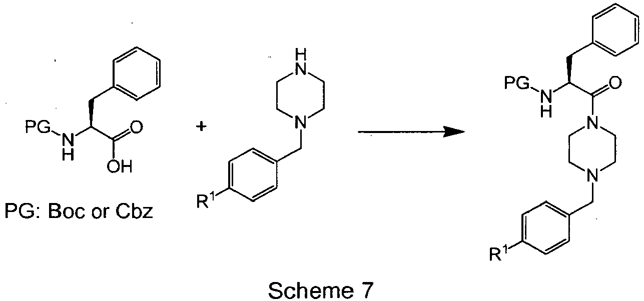

To a stirred suspension of 1 mmol of Boc-Phe-OH or Cbz-Phe-OH in 0.6 mL of dry DCM (or DMF) under nitrogen are successively added 1 mmol of TBTU and 2 mmol of NEM. The resulting light yellow suspension is stirred at RT for 1 h before a solution of 1 mmol of benzylpiperazine in 0.25 mL of dry DCM (or DMF) is added. The obtained reaction mixture is further stirred at RT overnight. Upon completion the reaction is diluted with DCM and quenched with a saturated solution of NaHCO₃. The aqueous phase is extracted with DCM (X3), the combined organic phases are successively washed with H₂O and brine, dried over Na₂SO₄, filtered and concentrated under reduced pressure. The residue is purified by flash chromatography (SiO₂ 60F) to afford the title compound.

### Intermediates 1

| R¹/PG | Chemical name | Yield | LC-MS | |
|---|---|---|---|---|
| | | | Rt (min) | [M+H]⁺ |
| H/Boc | (S)-[1-Benzyl-2-(4-benzyl-piperazin-1-yl)-2-oxo-ethyl]-carbamic acid tert-butyl ester | Quant. | 0.98* | 424.22 |
| CN/Boc | (S)-{1-Benzyl-2-[4-(4-cyano-benzyl)-piperazin-1-yl]-2-oxo-ethyl}-carbamic acid tert-butyl ester | 94% | 0.92* | 449.12 |
| N(CH₃)₂/ Boc | (S)-{1-Benzyl-2-[4-(4-dimethylamino-benzyl)-piperazin-1-yl]-2-oxo-ethyl}-carbamic acid tert-butyl ester | Quant. | 0.96* | 467.23 |
| Cl/Boc | (S)-{1-Benzyl-2-4-(4-chloro-benzyl)-piperazin-1-yl]-2-oxo-ethyl}-carbamic acid tert-butyl ester | Quant. | 1.01* | 457.79 |
| S(O₂)Me/ Cbz | (S)-Benzyl (1-(4-(4-(methylsulfonyl)benzyl)piperazin-1-yl)-1-oxo-3-phenylpropan-2-yl)carbamate | 76% | 0.62** | 536.16 |
| S(O₂)Et/ Cbz | (S)-Benzyl (1-(4-(4-(ethylsulfonyl)benzyl)piperazin-1-yl)-1-oxo-3-phenylpropan-2-yl)carbamate | 47% | 0.64** | 550.17 |

| | | | | |
|---|---|---|---|---|
| * Analytic I, X-Bridge column, basic conditions. ** Analytic, Waters Atlantis T3 column, acidic conditions | | | | |

### General Method B - step 2

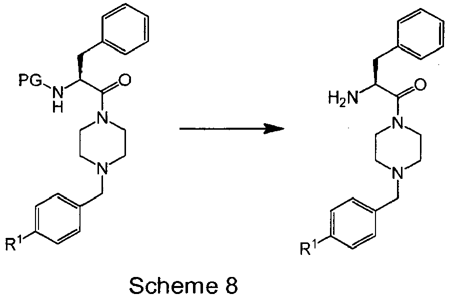

### With PG = Boc:

To a solution of 1 mmol of the Boc-protected amine in 9 mL of dry DCM at 0°C are added dropwise 4.5 mL of HCl 4N in dioxane. The resulting reaction mixture is stirred at RT for 4 h under nitrogen atmosphere, cooled down to 0°C and carefully neutralized to pH = 7 with an aqueous solution of NaOH 1 N. The aqueous phase is then extracted with DCM (X3). The combined organic phases are successively washed with H₂O and brine, dried over Na₂SO₄, filtered and concentrated under reduced pressure to afford the free primary amine, which is used in the next step without further purification.

### With PG = Cbz:

A mixture of 2 mmol of the Cbz-protected amine, Pd-C 10% (100 mg) in dry EtOH (25 mL) is stirred at RT under hydrogen atmosphere for 3 h. The reaction mixture is filtered over Celite and concentrated under reduced pressure to afford the free primary amine, which is used in the next step without further purification.

### Intermediates 2

| R¹ | Chemical name | Yield | LC-MS | |
|---|---|---|---|---|
| | | | Rt (min) | [M+H]⁺ |
| H | (S)-2-Amino-1-(4-benzyl-piperazin-1-yl)-3-phenyl-propan-1-one | Quant. | 0.79* | 324.29 |
| CN | (S)-4-[4-(2-Amino-3-phenyl-propionyl)-piperazin-1-ylmethyl]-benzonitrile | Quant. | 0.74* | 349.16 |
| N(CH₃)₂ | (S)-2-Amino-1-[4-(4-dimethylaminobenzyl)-piperazin-1yl]-3-phenyl-propan-1-one | Quant. | 0.78* | 367.19 |
| Cl | (S)-2-Amino-1-[4-(4-chloro-benzyl)-piperazin-1-yl]-3-phenyl-propan-1-one | Quant. | 0.82* | 358.11 |
| S(O₂)Me | (S)-2-Amino-1-(4-(4-(methylsulfonyl)benzyl)piperazin-1-yl)-3-phenylpropan-1-one | 89% | 0.37** | 401.79 |
| S(O₂)Et | (S)-2-Amino-1-(4-(4-(ethylsulfonyl)benzyl)piperazin-1-yl)-3-phenylpropan-1-one | 88% | 0.4** | 416.07 |

| | | | | |
|---|---|---|---|---|
| * Analytic I, X-Bridge column, basic conditions. ** Analytic, Waters Atlantis T3 column, acidic conditions | | | | |

### General Methods C1 & C2 - step 3

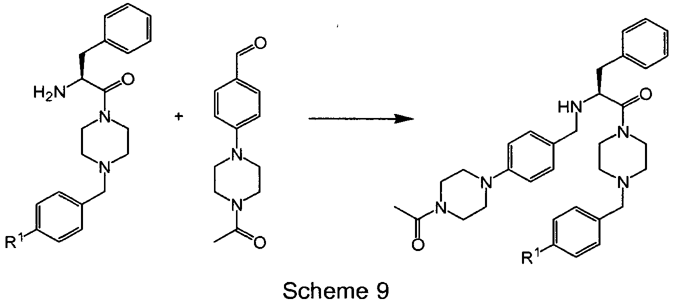

### General Method C1:

A solution of 1 mmol of amine and 1 mmol of aldehyde in 5 mL of dry MeOH is refluxed for 24 h under nitrogen. The resulting mixture is then cooled to RT prior to the addition of 1.5 mmol of NaBH₄ in portion. The obtained heterogeneous mixture is further stirred for 2 h at RT, quenched with a saturated aqueous solution of NaHCO₃ and extracted with EtOAc (X3). The combined organic phases are washed with brine, dried over Na₂SO₄, filtered and concentrated under reduced pressure. The residue is purified by flash chromatography (SiO₂ 60F) to afford the title compound.

### General Method C2:

To a solution of 1 mmol of amine and 1 mmol of aldehyde in 5 mL of dry CH₃CN are added portionwise 1.5 mmol of NaBH(OAc)₃. The resulting heterogeneous mixture is further stirred for 4 h at RT, quenched with a saturated aqueous solution of NaHCO₃ and extracted with EtOAc (X3). The combined organic phases are washed with brine, dried over Na₂SO₄, filtered and concentrated under reduced pressure. The residue is purified by flash chromatography (SiO₂ 60F) to afford the title compound.

### Intermediates 3

| R¹ | Chemical name | Yield | LC-MS | |
|---|---|---|---|---|
| | | | Rt (min) | [M+H]⁺ |
| H | (S)-2-[4-(4-Acetyl-piperazin-1-yl)-benzylamino]-1-(4-benzyl-piperazin-1-yl)-3-pnenyl-propan-1-one | 77% | 0.84* | 540.30 |
| CN | (S)-4-(4-{2-[4-(4-Acetyl-piperazin-1-yl)-benzylamino]-3-phenyl-propionyl}-piperazin-1-ylmethyl)-benzonitrile | 81% | 0.79* | 565.16 |
| N(CH₃)₂ | (S)-2-[4-(4-Acetyl-piperazin-1-yl)-benzylamino]-1-[4-(4-dimethylamino-benzyl)-piperazin-1-yl]-3-phenyl-propan-1-one | 64% | 0.83* | 583.20 |
| Cl | (S)-2-[4-(4-Acetyl-piperazin-1-yl)-benzylamino]-1-[4-(4-chloro-benzyl)-piperazin-1-yl]-3-phenyl-propan-1-one | 76% | 0.88* | 574.10 |
| S(O₂)Me | (S)-2-[4-(4-Acetyl-piperazin-1-yl)-benzylamino]-1-[4-(4-methylsulfonyl-benzyl)-piperazin-1-yl]-3-phenyl-propan-1-one | 52% | 0.43** | 618.22 |
| S(O₂)Et | (S)-2-[4-(4-Acetyl-piperazin-1-yl)-benzylamino]-1-[4-(4-ethylsulfonyl-benzyl)-piperazin-1-yl]-3-phenyl-propan-1-one | 80% | 0.45** | 632.2 |

| | | | | |
|---|---|---|---|---|
| * Analytic I, X-Bridge column, basic conditions. ** Analytic, Waters Atlantis T3 column, acidic conditions | | | | |

### General Methods D1 and D2 - step 4

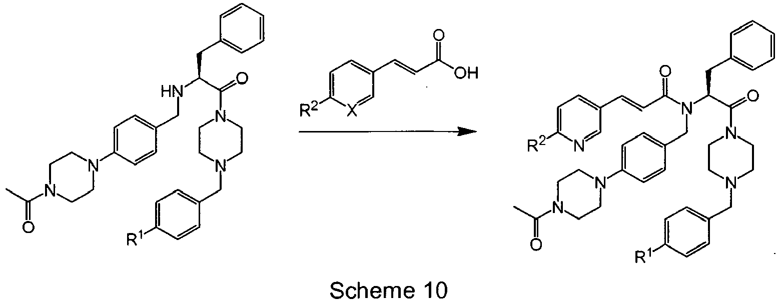

### General Method D1:

To a solution of 1 mmol of cinnamic acid in 5 mL of dry DCM under nitrogen are added 1.4 mmol of 1-chloro-N,N-2-trimethylpropenylamine (Ghosez's reagent). The resulting mixture is stirred at RT for 1 h before a solution of 1 mmol of amine and 3 mmol of DIPEA in 4 mL of dry DCM is added. The reaction mixture is further stirred at RT overnight. Upon completion a saturated aqueous solution of NaHCO₃ is added and the mixture extracted with DCM (X3). The combined organic phases are washed with brine, dried over Na₂SO₄, filtered and concentrated under reduced pressure. The residue is purified either by flash chromatography (SiO₂ 60F) or by preparative HPLC to afford the final compound.

### General Method D2:

To a solution (or suspension) of 1.05 mmol of cinnamic acid in 3.5 mL of dry DCM under nitrogen at 0°C are added 1.1 mmol of oxalyl chloride and 3 drops of DMF. The resulting mixture is stirred at RT for 1 h, cooled down to 0°C before a solution of 1 mmol of amine and 2 mmol of DIPEA in 3 mL of dry DCM is added. The reaction mixture is further stirred at RT overnight. Upon completion a saturated aqueous solution of NaHCO₃ is added and the mixture extracted with DCM (X3). The combined organic phases are washed with brine, dried over Na₂SO₄, filtered and concentrated under reduced pressure. The residue is purified either by flash chromatography (SiO₂ 60F) or by preparative HPLC to afford the final compound.

| Compound of Example # | Chemical name | LC-MS | |
|---|---|---|---|
| | | Rt (min) | [M+H]⁺ |
| 1** | (S)-N-[4-(4-Acetyl-piperazin-1-yl)-benzyl]-N-[1-benzyl-2-(4-benzyl-piperazin-1-yl)-2-oxo-ethyl]-3-(4-trifluoromethyl-phenyl)-acrylamide | 1.00* | 738.37 |
| 2 | (S)-N-[4-(4-Acetyl-piperazin-1-yl)-benzyl]-N-[1-benzyl-2-(4-benzyl-piperazin-1-yl)-2-oxo-ethyl]-3-(4-ethyl-phenyl)-acrylamide | 1.02* | 698.45 |
| 3 | (S)-N-[4-(4-Acetyl-piperazin-1-yl)-benzyl]-N-[1-benzyl-2-(4-benzyl-piperazin-1-yl)-2-oxo-ethyl]-3-(4-tert-butyl-phenyl)-acrylamide | 1.06* | 726.46 |
| 4 | (S)-N-[4-(4-Acetyl-piperazin-1-yl)-benzyl]-N-[1-benzyl-2-(4-benzyl-piperazin-1-yl)-2-oxo-ethyl]-3-(4-ethoxy-phenyl)-acrylamide | 0.98* | 714.26 |
| 5 | (S)-4-(2-{[4-(4-Acetyl-piperazin-1-yl)-benzyl]-[1-benzyl-2-(4-benzyl-piperazin-1-yl)-2-oxo-ethyl]-carbamoyl}-vinyl)-benzoic acid methyl ester | 0.94* | 728.39 |
| 6 | (S)-N-[4-(4-Acetyl-piperazin-1-yl)-benzyl]-N-[1-benzyl-2-(4-benzyl-piperazin-1-yl)-2-oxo-ethyl]-3-(4-methanesulfonyl-phenyl)-acrylamide | 0.88* | 748.36 |
| 7 | (S)-3-(4-Acetylamino-phenyl)-N-[4-(4-acetyl-piperazin-1-yl)-benzyl]-N-[1-benzyl-2-(4-benzyl-piperazin-1-yl)-2-oxo-ethyl]-acrylamide | 0.86* | 727.37 |
| 8 | (S)-N-[4-(4-Acetyl-piperazin-1-yl)-benzyl]-N-[1-benzyl-2-(4-benzyl-piperazin-1-yl)-2-oxo-ethyl]-3-(4-propoxy-phenyl)-acrylamide | 1.02* | 728.40 |
| 9 | (S)-N-[4-(4-Acetyl-piperazin-1-yl)-benzyl]-N-[1-benzyl-2-(4-benzyl-piperazin-1-yl)-2-oxo-ethyl]-3-(4-isopropoxy-phenyl)-acrylamide | 1.00* | 728.43 |
| 10 | (S)-N-[4-(4-Acetyl-piperazin-1-yl)-benzyl]-N-[1-benzyl-2-(4-benzyl-piperazin-1-yl)-2-oxo-ethyl]-3-(4-isopropyl-phenyl)-acrylamide | 1.05* | 712.28 |
| 11 | (S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-trifluoromethyl-phenyl)-acrylamide | 0.99* | 781.18 |
| 12 | (S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(6-trifluoromethyl-pyridin-3-yl)-acrylamide | 0.94* | 782.19 |
| 13 | (S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(6-methoxy-pyridin-3-yl)-acrylamide | 0.91* | 744.20 |
| 14 | (S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(6-ethoxy-pyridin-3-yl)-acrylamide | 0.94* | 758.22 |
| 15 | (S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-ethoxy-phenyl)-acrylamide | 0.97* | 757.17 |
| 16 | (S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-methanesulfonyl-phenyl)-acrylamide | 0.87* | 791.15 |
| 17 | (S)-3-(4-Acetylamino-phenyl)-N-[4-(4-acetyl-piperazin-1-yl)-benzyl]-N-{1-benzyl-2-[4-(4-dimethylamino-benzyl)-piperazin-1-yl]-2-oxo-ethyl}-acrylamide | 0.85* | 770.22 |
| 18 | (S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-propoxy-phenyl)-acrylamide | 1.00* | 771.27 |
| 19 | (S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-isopropoxy-phenyl)-acrylamide | 0.99* | 771.25 |
| 20 | (S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-ethyl-phenyl)-acrylamide | 1.00* | 741.28 |
| 21 | (S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-difluoromethoxy-phenyl)-acrylamide | 0.95* | 779.15 |
| 22 | (S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-tert-butyl-phenyl)-acrylamide | 1.05* | 769.26 |
| 23 | (S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-isopropyl-phenyl)-acrylamide | 1.03* | 755.28 |
| 24 | (S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-trifluoromethyl-phenyl)-acrylamide | 0.96* | 763.11 |
| 25 | (S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-ethoxy-phenyl)-acrylamide | 0.93* | 739.22 |
| 26 | (S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-methanesulfonyl-phenyl)-acrylamide | 0.84* | 773.00 |
| 27 | (S)-3-(4-Acetylamino-phenyl)-N-[4-(4-acetyl-piperazin-1-yl)-benzyl]-N-{1-benzyl-2-[4-(4-cyanobenzyl)-piperazin-1-yl]-2-oxo-ethyl}-acrylamide | 0.82* | 752.02 |
| 28 | (S)-N-[4-(4-Acetyl-piperazin-1-yl)-benzyl]-N-{1-benzyl-2-[4-(4-cyano-benzyl)-piperazin-1-yl]-2-oxoethyl}-3-(4-propoxy-phenyl)-acrylamide | 0.97* | 753.15 |
| 29 | (S)-N-[4-(4-Acetyl-piperazin-1-yl)-benzyl]-N-{1-benzyl-2-[4-(4-cyano-benzyl)-piperazin-1-yl]-2-oxoethyl}-3-(4-isopropoxy-phenyl)-acrylamide | 0.96* | 753.18 |
| 30 | (S)-N-[4-(4-Acetyl-piperazin-1-yl)-benzyl]-N-{1-benzyl-2-[4-(4-cyano-benzyl)-piperazin-1-yl]-2-oxoethyl}-3-(4-ethyl-phenyl)-acrylamide | 0.97* | 723.19 |
| 31 | (S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-difluoromethoxy-phenyl)-acrylamide | 0.92* | 761.06 |
| 32 | (S)-N-[4-(4-Acetyl-piperazin-1-yl)-benzyl]-N-{1-benzyl-2-[4-(4-cyano-benzyl)-piperazin-1-yl]-2-oxoethyl}-3-(4-isopropyl-phenyl)-acrylamide | 0.99* | 737.23 |
| 33 | (S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-trifluoromethoxy-phenyl)-acrylamide | 0.97* | 778.97 |
| 34 | (S)-N-[4-(4-Acetyl-piperazin-1-yl)-benzyl]-N-{1-benzyl-2-[4-(4-cyano-benzyl)-piperazin-1-yl]-2-oxoethyl}-3-(4-tert-butyl-phenyl)-acrylamide | 1.02* | 751.21 |
| 35 | (S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-ethoxy-phenyl)-acrylamide | 1.00* | 748.13 |
| 36 | (S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-methanesulfonyl-phenyl)-acrylamide | 0.90* | 782.11 |
| 37 | (S)-3-(4-Acetylamino-phenyl)-N-[4-(4-acetyl-piperazin-1-yl)-benzyl]-N-{1-benzyl-2-[4-(4-chlorobenzyl)-piperazin-1-yl]-2-oxo-ethyl}-acrylamide | 0.88* | 761.18 |
| 38 | (S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-propoxy-phenyl)-acrylamide | 1.03* | 762.18 |
| 39 | (S)-N-[4-(4-Acetyl-piperazin-1-yl)-benzyl]-N-{1-benzyl-2-[4-(4-chloro-benzyl)-piperazin-1-yl]-2-oxoethyl}-3-(4-isopropoxy-phenyl)-acrylamide | 1.02* | 762.13 |
| 40 | (S)-N-[4-(4-Acetyl-piperazin-1-yl)-benzyl]-N-{1-benzyl-2-[4-(4-chloro-benzyl)-piperazin-1-yl]-2-oxoethyl}-3-(4-ethyl-phenyl)-acrylamide | 1.04* | 732.25 |
| 41 | (S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-difluoromethoxy-phenyl)-acrylamide | 0.98* | 770.15 |
| 42 | (S)-N-[4-(4-Acetyl-piperazin-1-yl)-benzyl]-N-{1-benzyl-2-[4-(4-chloro-benzyl)-piperazin-1-yl]-2-oxoethyl}-3-(4-isopropyl-phenyl)-acrylamide | 1.06* | 745.96 |
| 43 | (S)-N-[4-(4-Acetyl-piperazin-1-yl)-benzyl]-N-{1-benzyl-2-[4-(4-chloro-benzyl)-piperazin-1-yl]-2-oxoethyl}-3-(4-tert-butyl-phenyl)-acrylamide | 1.08* | 760.13 |
| 44 | (S)-N-[4-(4-Acetyl-piperazin-1-yl)-benzyl]-N-{1-benzyl-2-[4-(4-(methylsulfonyl)-benzyl)-piperazin-1-yl]-2-oxo-ethyl}-3-(4-isopropoxyphenyl)-acrylamide | 0.73*** | 806.37 |
| 45 | (S)-N-[4-(4-Acetyl-piperazin-1-yl)-benzyl]-N-{1-benzyl-2-[4-(4-(methylsulfonyl)-benzyl)-piperazin-1-yl]-2-oxo-ethyl}-3-(4-tert-butyl-phenyl)-acrylamide | 0.78*** | 804.37 |
| 46 | (S)-N-[4-(4-Acetyl-piperazin-1-yl)-benzyl]-N-{1-benzyl-2-[4-(4-(methylsulfonyl)-benzyl)-piperazin-1-yl]-2-oxo-ethyl}-3-(4-propoxyphenyl)-acrylamide | 0.76*** | 806.37 |
| 47 | (S)-N-(4-(4-acetylpiperazin-1-yl)benzyl)-N-(1-(4-(4-(methylsulfonyl)benzyl)piperazin-1-yl)-1-oxo-3-phenylpropan-2-yl)-3-(4-(trifluoromethyl)phenyl)acrylamide | 0.75*** | 816.23 |
| 48 | (S)-N-[4-(4-Acetyl-piperazin-1-yl)-benzyl]-N-{1-benzyl-2-[4-(4-(ethylsulfonyl)-benzylypiperazin-1-yl]-2-oxo-ethyl}-3-(4-propoxyphenyl)-acrylamide | 0.77*** | 820.43 |
| 49 | (S)-N-(4-(4-acetylpiperazin-1-yl)benzyl)-N-(1-(4-(4-(ethylsulfonyl)benzyl)piperazin-1-yl)-1-oxo-3-phenylpropan-2-yl)-3-(4-(trifluoromethyl)phenyl)acrylamide | 0.76*** | 829.85 |

| | | | |
|---|---|---|---|
| * Analytic I, X-Bridge column, basic conditions. ** Reference Example *** Analytic, Waters Atlantis T3 column, acidic conditions | | | |

### Preparation of compounds of formula I via pathway depicted in Scheme 2 - General Procedures and Examples:

### Step 1: (S)-[1-Benzyl-2-(4-{4-[(2-hydroxy-ethyl)-methyl-amino]-benzyl}-piperazin-1-yl)-2-oxo-ethyl]-carbamic acid tert-butyl ester.

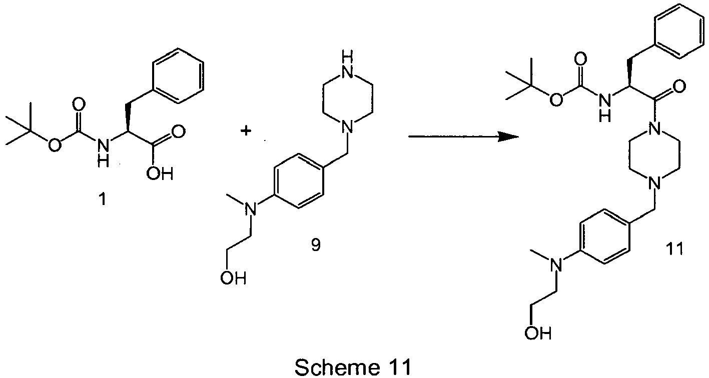

According to general Method A, 60 mmol of Boc-Phe-OH are used to provide the title compound in 50% yield. Rt = 0.87; [M+H]⁺ = 497.42 (Analytic I, X-Bridge column, basic conditions).

### Step 2: (S)-2-Amino-1-(4-{4-[(2-hydroxy-ethyl)-methyl-amino]-benzyl}-piperazin-1-yl)-3-phenyl-propan-1-one.

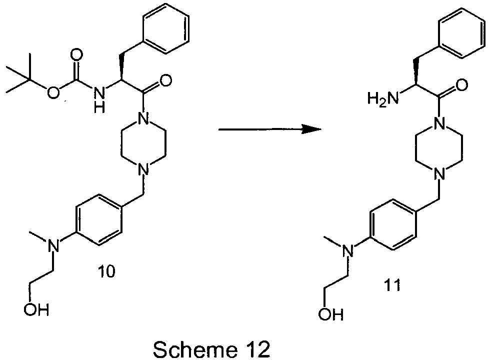

According to general Method B, 12 mmol of Boc-protected amine 10 are used to provide the title compound in quantitative yield. Rt = 0.69; [M+H]⁺ = 397.18 (Analytic I, X-Bridge column, basic conditions).

### Step 3: (S)-2-[4-(4-Acetyl-piperazin-1-yl)-benzylamino]-1-(4-{4-[(2-hydroxy-ethyl)-methyl-amino]-benzyl}-piperazin-1-yl)-3-phenyl-propan-1-one.

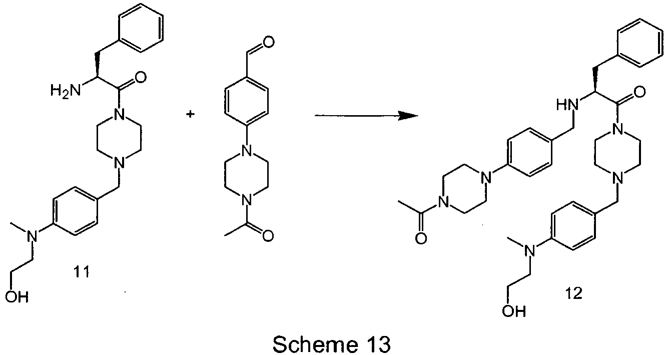

According to general Method C2, 11.4 mmol of free amine 11 are used to provide the title compound in quantitative yield. Rt = 0.74; [M+H]⁺ = 613.24 (Analytic I, X-Bridge column, basic conditions).

### General Method E - step 4: (S)-2-[4-(4-Acetyl-piperazin-1-yl)-benzylamino]-1-[4-(4-{[2-(tert-butyl-dimethyl-silanyloxy)-ethyl]-methyl-amino}-benzyl)-piperazin-1-yl]-3-phenyl-propan-1-one.

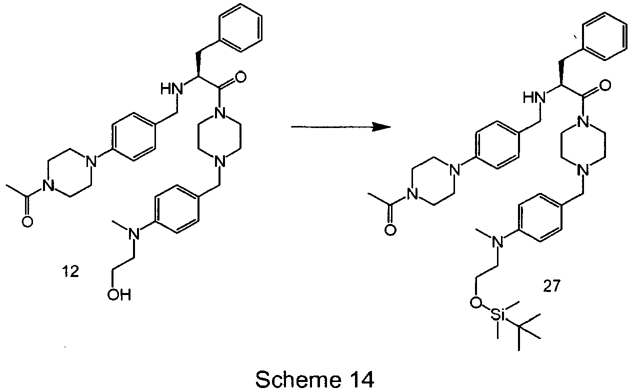

3 mmol of TBDMSCI are added portionwise to a solution of 1 mmol of hydroxyl 12 and 3 mmol of imidazole in 5 mL of dry DMF at RT. The yellow solution is stirred at RT for 16 h, quenched with H₂O and extracted with EtOAc (X3). The combined organic phases are washed with brine, dried over Na₂SO₄, filtered and concentrated under reduced pressure. The residue is purified by flash chromatography (SiO₂ 60F; DCM/MeOH 92:8 to 95:5) to afford the title compound as yellow foam in 81% yield. Rt = 1.12 min; [M+H]⁺ = 727.31. (Analytic I, X-Bridge column, basic conditions).

### Step 5

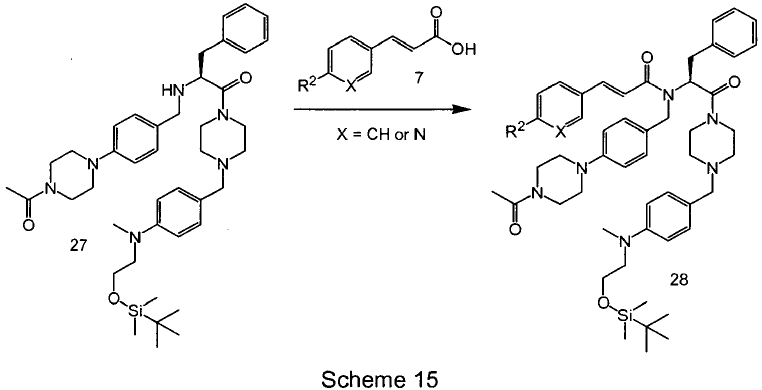

The compounds of formula 28 are obtained according to general Methods D1 or D2.

| Intermediate # | Chemical name | LC-MS* | |
|---|---|---|---|
| | | Rt (min) | [M+H]⁺ |
| 4 | (S)-N-[4-(4-Acetyl-piperazin-1-yl)-benzyl]-N-{1-benzyl-2-[4-(4-{[2-(tert-butyl-dimethyl-silanyloxy)-ethyl]-methyl-amino}-benzyl)-piperazin-1-yl]-2-oxo-ethyl}-3-(4-trifluoromethyl-phenyl)-acrylamide | 1.21 | 925.21 |
| 5 | (S)-N-[4-(4-Acetyl-piperazin-1-yl)-benzyl]-N-{1-benzyl-2-[4-(4-{[2-(tert-butyl-dimethyl-silanyloxy)-ethyl]-methyl-amino}-benzyl)-piperazin-1-yl]-2-oxoethyl}-3-(6-trifluoromethyl-pyridin-3-yl)-acrylamide | 1.15 | 926.27 |
| 6 | (S)-N-[4-(4-Acetyl-piperazin-1-yl)-benzyl]-N-{1-benzyl-2-[4-(4-{[2-(tert-butyl-dimethyl-silanyloxy)-ethyl]-methyl-amino}-benzyl)-piperazin-1-yl]-2-oxoethyl}-3-(6-methoxy-pyridin-3-yl)-acrylamide | 1.14 | 888.29 |
| 7 | (S)-N-[4-(4-Acetyl-piperazin-1-yl)-benzyl]-N-{1-benzyl-2-[4-(4-{[2-(tert-butyl-dimethyl-silanyloxy)-ethyl]-methyl-amino}-benzyl)-piperazin-1-yl]-2-oxo-ethyl}-3-(6-ethoxy-pyridin-3-yl)-acrylamide | 1.19 | 902.33 |
| 8 | (S)-N-[4-(4-Acetyl-piperazin-1-yl)-benzyl]-N-{1-benzyl-2-[4-(4-{[2-(tert-butyl-dimethyl-silanyloxy)-ethyl]-methyl-amino}-benzyl)-piperazin-1-yl]-2-oxo-ethyl}-3-(4-ethoxy-phenyl)-acrylamide | 1.20 | 901.29 |
| 9 | (S)-N-[4-(4-Acetyl-piperazin-1-yl)-benzyl]-N-{1-benzyl-2-[4-(4-{[2-(tert-butyl-dimethyl-silanyloxy)-ethyl]-methyl-amino}-benzyl)-piperazin-1-yl]-2-oxo-ethyl}-3-(4-methanesulfonyl-phenyl)-acrylamide | 1.09 | 935.21 |
| 10 | (S)-3-(4-Acetylamino-phenyl)-N-[4-(4-acetyl-piperazin-1-yl)-benzyl]-N-{1-benzyl-2-[4-(4-{[2-(tert-butyl-dimethyl-silanyloxy)-ethyl]-methyl-amino}-benzyl)-piperazin-1-yl]-2-oxo-ethyl}-acrylamide | 1.08 | 914.29 |
| 11 | (S)-N-[4-(4-Acetyl-piperazin-1-yl)-benzyl]-N-{1-benzyl-2-[4-(4-{[2-(tert-butyl-dimethyl-silanyloxy)-ethyl]-methyl-amino}-benzyl)-piperazin-1-yl]-2-oxoethyl}-3-(4-propoxy-phenyl)-acrylamide | 1.24 | 915.28 |
| 12 | (S)-N-[4-(4-Acetyl-piperazin-1-yl)-benzyl]-N-{1-benzyl-2-[4-(4-{[2-(tert-butyl-dimethyl-silanyloxy)-ethyl]-methyl-amino}-benzyl)-piperazin-1-yl]-2-oxo-ethyl}-3-(4-isopropoxy-phenyl)-acrylamide | 1.22 | 915.32 |
| 13 | (S)-N-[4-(4-Acetyl-piperazin-1-yl)-benzyl]-N-{1-benzyl-2-[4-(4-{[2-(tert-butyl-dimethyl-silanyloxy)-ethyl]-methyl-amino}-benzyl)-piperazin-1-yl]-2-oxo-ethyl}-3-(4-ethyl-phenyl)-acrylamide | 1.25 | 885.35 |
| 14 | (S)-N-[4-(4-Acetyl-piperazin-1-yl)-benzyl]-N-{1-benzyl-2-[4-(4-{[2-(tert-butyl-dimethyl-silanyloxy)-ethyl]-methyl-amino}-benzyl)-piperazin-1-yl]-2-oxo-ethyl}-3-(4-difluoromethoxy-phenyl)-acrylamide | 1.15 | 923.21 |
| 15 | (S)-N-[4-(4-Acetyl-piperazin-1-yl)-benzyl]-N-{1-benzyl-2-[4-(4-{[2-(tert-butyl-dimethyl-silanyloxy)-ethyl]-methyl-amino}-benzyl)-piperazin-1-yl]-2-oxo-ethyl}-3-(4-isopropyl-phenyl)-acrylamide | 1.29 | 899.32 |
| 16 | (S)-N-[4-(4-Acetyl-piperazin-1-yl)-benzyl]-N-{1-benzyl-2-[4-(4-{[2-(tert-butyl-dimethyl-silanyloxy)-ethyl]-methyl-amino}-benzyl)-piperazin-1-yl]-2-oxoethyl}-3-(4-tert-butyl-phenyl)-acrylamide | 1.32 | 913.29 |

| | | | |
|---|---|---|---|
| * Analytic I, X-Bridge column, basic conditions. | | | |

### General Method F - Step 6

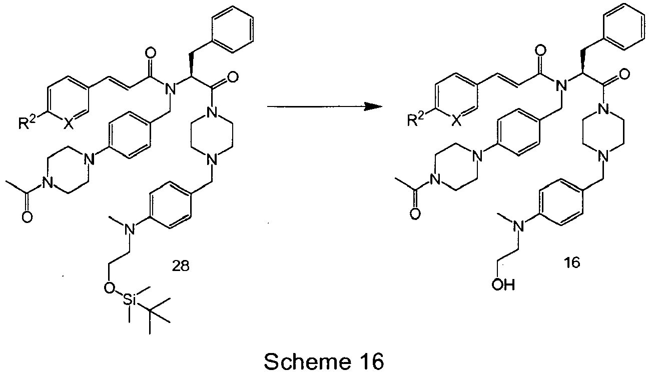

To a solution of 1 mmol of protected alcohol **28** in 3.5 mL of dry THF under nitrogen at 0°C are added 3.5 mmol of TBAF (1 M solution in THF). The resulting mixture is stirred at RT for 4 h and cooled down to 0°C. Upon completion H₂O is added and the mixture extracted with DCM (X3). The combined organic phases are washed with brine, dried over Na₂SO₄, filtered and concentrated under reduced pressure. The residue is purified either by flash chromatography (SiO₂ 60F) or by preparative HPLC to afford the final compound.

| Compound of Example # | Chemical name | LC-MS* | |
|---|---|---|---|
| | | Rt (min) | [M+H]⁺ |
| 50 | (S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-trifluoromethyl-phenyl)-acrylamide | 0.91 | 811.21 |
| 51 | (S)-N-[4-(4-Acetyl-piperazin-1-yl)-benzyl]-N-[1-benzyl-2-(4-{4-[(2-hydroxy-ethyl)methyl-amino]-benzyl}-piperazin-1-yl)-2-oxo-ethyl]-3-(6-trifluoromethyl-pyridin-3-yl)-acrylamide | 0.86 | 812.24 |
| 52 | (S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(6-methoxy-pyridin-3-yl)-acrylamide | 0.82 | 774.25 |
| 53 | (S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(6-ethoxy-pyridin-3-yl)-acrylamide | 0.85 | 788.24 |
| 54 | (S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-ethoxyphenyl)-acrylamide | 0.88 | 787.19 |
| 55 | (S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-methanesulfonyl-phenyl)-acrylamide | 0.80 | 821.18 |
| 56 | (S)-3-(4-Acetylamino-phenyl)-N-[4-(4-acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-acrylamide | 0.77 | 800.21 |
| 57 | (S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-propoxy-phenyl)-acrylamide | 0.92 | 801.22 |
| 58 | (S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-isopropoxy-phenyl)-acrylamide | 0.91 | 801.25 |
| 59 | (S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-ethyl-phenyl)-acrylamide | 0.92 | 771.29 |
| 60 | (S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-difluoromethoxy-phenyl)-acrylamide | 0.87 | 808.87 |
| 61 | (S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-isopropyl-phenyl)-acrylamide | 0.94 | 787.34 |
| 62 | (S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-tert-butyl-phenyl)-acrylamide | 0.97 | 799.22 |

| | | | |
|---|---|---|---|
| * Analytic I, X-Bridge column, basic conditions. | | | |

### Preparation of compounds of formula I via pathway depicted in Scheme 3 - General Procedures and Examples:

### Step 1: (S)-2-[4-(4-Acetyl-piperazin-1-yl)-benzylamino]-3-phenyl-propionic acid methyl ester.

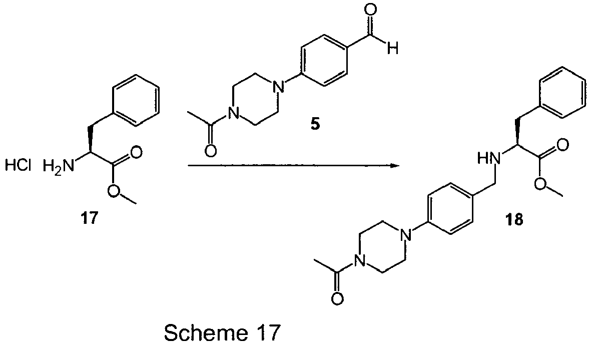

The secondary amine 18 is obtained according to general Methods C1 or C2 and used in the next step without further purification. Rt = 0.82; [M+H]⁺ = 396.20 (Analytic I, X-Bridge column, basic conditions).

### Step 2

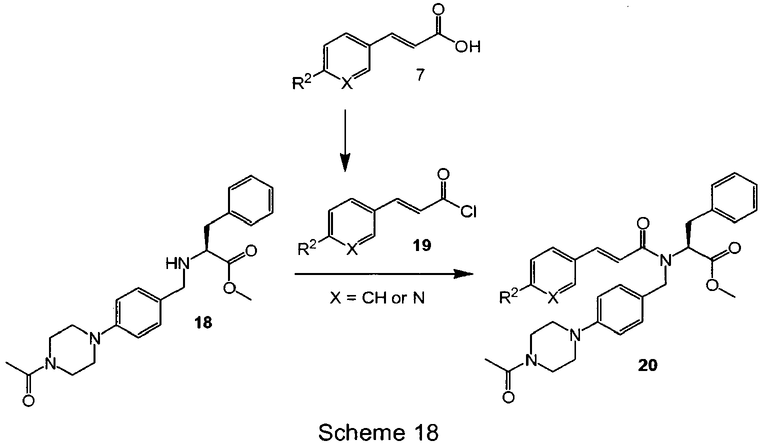

The compounds of formula 20 are obtained according to general Methods D1 or D2.

| Intermediate # | Chemical name | Yield | LC-MS | | |
|---|---|---|---|---|---|
| | | | t_{R} (min) | [M+H]⁺ | conditions |
| 17 | (S)-2-{[4-(4-Acetyl-piperazin-1-yl)-benzyl]-[3-(4-trifluoromethyl-phenyl)-acryloyl]-amino}-3-phenyl-propionic acid methyl ester | 70% | 0.96 | 594.15 | Analytic I, X-Bridge column, basic conditions |

### General Method G - Step 3

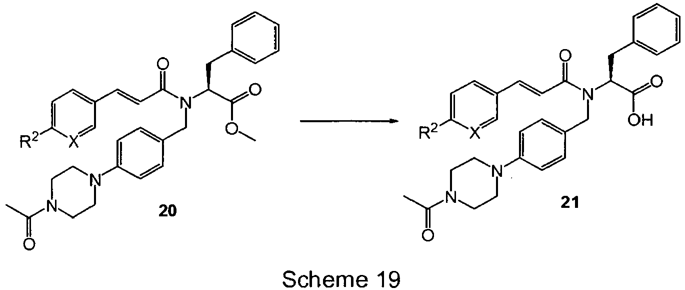

To a solution of 1 mmol of methyl ester 20 in 8 mL of Et₂O and 2 mL of H₂O at 0°C are added dropwise 10 mmol of a 2M aqueous NaOH. The reaction mixture is further stirred at RT for 2-3 h. Upon completion the aqueous phase is acidified to pH = 2-3 with HCl 1 N and then extracted with EtOAc (X3). The combined organic phases are washed with brine, dried over Na₂SO₄, filtered and concentrated under reduced pressure. The residue is used in the next step without further purification.

| Intermediate # | Chemical name | Yield | LC-MS | | |
|---|---|---|---|---|---|
| | | | t_{R} (min) | [M+H]⁺ | conditions |
| 18 | (S)-2-{[4-(4-Acetyl-piperazin-1-yl)-benzyl]-[3-(4-trifluoromethyl-phenyl)-acryloyl]-amino}-3-phenyl-propionic acid | 78% | 0.97 | 580.12 | Analytic I, X-Bridge column, basic conditions |

### Step 4

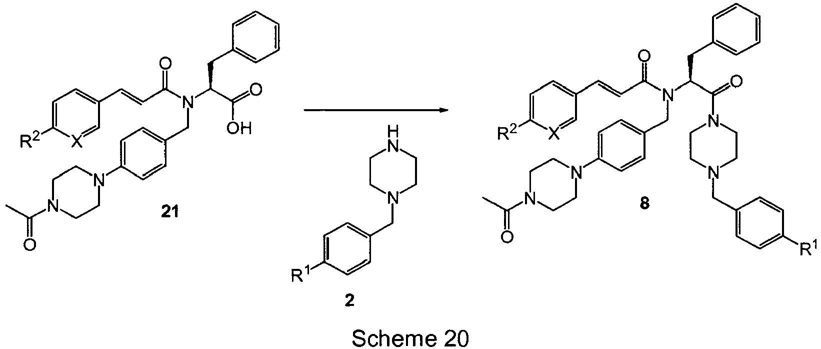

The compounds of formula **8** are obtained according to general Method A. The residue is purified either by flash chromatography (SiO₂ 60F) or by preparative HPLC to afford the final compound.

| Compound of Example # | Chemical name | LC-MS* | |
|---|---|---|---|
| | | Rt (min) | [M+H]⁺ |
| 63 | (S)-N-[4-(4-Acetyl-piperazin-1-yl)-benzyl]-N-{1-benzyl-2-[4-(4-methoxy-benzyl)-piperazin-1-yl]-2-oxo-ethyl}-3-(4-trifluoromethyl-phenyl)-acrylamide | 0.99 | 768.24 |
| 64 | (S)-N-[4-(4-Acetyl-piperazin-1-yl)-benzyl]-N-{1-benzyl-2-[4-(4-nitro-benzyl)-piperazin-1-yl]-2-oxo-ethyl}-3-(4-trifluoromethyl-phenyl)-acrylamide | 0.99 | 783.22 |
| 65 | (S)-N-[4-(4-Acetyl-piperazin-1-yl)-benzyl]-N-{1-benzyl-2-[4-(4-isopropoxy-benzyl)-piperazin-1-yl]-2-oxo-ethyl}-3-(4-trifluoromethyl-phenyl)-acrylamide | 1.02 | 796.24 |

| | | | |
|---|---|---|---|
| * Analytic I, X-Bridge column, basic conditions. | | | |

The compounds of Examples 66 to 73 are obtained according to general Method D2.

| Compound of Example # | Chemical name | LC-MS* | |
|---|---|---|---|
| | | Rt (min) | [M+H]⁺ |
| 66 | (S)-N-[4-(4-Acetyl-piperazin-1-yl)benzyl]-N-{1-(4-(4-(dimethylamino)benzyl)piperazin-1-yl)-1-oxo-3-phenylpropan-2-yl}cinnamamide | 0.94 | 713.13 |
| 67 | (S)-N-[4-(4-Acetyl-piperazin-1-yl)benzyl]-N-{1-[4-(4-(dimethylamino)benzyl)piperazin-1-yl]-1-oxo-3-phenylpropan-2-yl}-3-(p-tolyl)acrylamide | 0.97 | 727.45 |
| 68 | (S)-N-[4-(4-Acetyl-piperazin-1-yl)benzyl]-N-{1-(4-(4-(dimethylamino)benzyl)piperazin-1-yl)-1-oxo-3-phenylpropan-2-yl}-3-(4-propylphenyl)acrylamide | 1.04 | 755.45 |
| 69 | (S)-N-[4-(4-Acetyl-piperazin-1-yl)benzyl]-N-{1-(4-(4-(dimethylamino)benzyl)piperazin-1-yl)-1-oxo-3-phenylpropan-2-yl}-3-(4-methoxyphenyl)acrylamide | 0.93 | 743.46 |
| 70 | (S)-3-(4-Acetyl-phenyl)-N-[4-(4-acetyl-piperazin-1-yl)benzyl]-N-{1-(4-(4-(dimethylamino)benzyl)piperazin-1-yl)-1-oxo-3-phenylpropan-2-yl}acrylamide | 0.91 | 755.43 |
| 71 | (S)-N-[4-(4-Acetyl-piperazin-1-yl)benzyl]-3-(4-cyanophenyl)-N-{1-(4-(4-(dimethylamino)benzyl)piperazin-1-yl)-1-oxo-3-phenylpropan-2-yl}acrylamide | 0.92 | 738.44 |
| 72 | (S)-N-[4-(4-Acetyl-piperazin-1-yl)benzyl]-N-{1-(4-(4-(dimethylamino)benzyl)piperazin-1-yl)-1-oxo-3-phenylpropan-2-yl}-3-(4-fluorophenyl)acrylamide | 0.95 | 731.42 |
| 73 | (S)-N-[4-(4-Acetyl-piperazin-1-yl)benzyl]-3-(4-chlorophenyl)-N-{1-(4-(4-(dimethylamino)benzyl)piperazin-1-yl)-1-oxo-3-phenylpropan-2-yl}acrylamide | 0.98 | 747.28 |

| | | | |
|---|---|---|---|
| * Analytic I, X-Bridge column, basic conditions. | | | |

### In vitro antimalarial activity: Plasmodium falciparum in vitro assay

*In vitro* activity against erythrocytic stages of P. falciparum in human red blood cells is determined using a [³H] hypoxanthine incorporation assay. One strain sensitive to all known drugs (P. falciparum NF54) is used in this assay and all tested compounds are compared for activity with the standard drugs chloroquine (sigma C6628) and artesunate (sigma 36, 159-3). Compounds, tested in duplicates, are serially diluted with screening medium [RPMI 1640 medium, supplemented with HEPES (5.94 g/L), NaHCO₃ (2.1 g/L), neomycin (100 U/mL), and Albumax (5 g/L) or human serum (50% final concentration)] in 96-well microtiter plates within an appropriate concentration range. Thereafter, the parasite cultures incubated in screening medium containing washed human red blood cells at 2.5% hematocrit (0.3% parasitemia) are added to the serially diluted compounds and incubated in a humidifying atmosphere at 37°C, 4% CO₂, 3% O₂, and 93% N₂. After 48 h, [³H] hypoxanthine (0.5 µCi) is added to each well of a plate. The plates are incubated for a further 24 h under the same conditions then harvested with a Betaplate cell harvester (Wallac) and washed with distilled water. The dried filters are inserted into a plastic foil with 10 mL of scintillation fluid, and counted in a Betaplate liquid scintillation counter. IC₅₀ values are calculated from sigmoidal inhibition curves using Microsoft Excel.

**Table 1: IC₅₀ values (nM) for compounds of formula I:**

| **Compound of Example #** | **IC₅₀ on NF54 (with Albumax)** | **IC₅₀ on NF54 (with 50% Serum)** |
|---|---|---|
| **1** (Reference Example) | 5.1 | 27.6 |
| **2** | 2.0 | 10 |
| **3** | 1.9 | 8.5 |
| **4** | 1.9 | 10 |
| **5** | 3.9 | 12 |
| **6** | 9.4 | 32 |
| **7** | 3.1 | 40 |
| **8** | 1.8 | 13.4 |
| **9** | 2.4 | 7.3 |
| **10** | 3.0 | 8.6 |
| **11** | 4.0 | 21 |
| **12** | 17 | 74 |
| **13** | 12 | 46 |
| **14** | 3.7 | 20 |
| **15** | 1.7 | 6.8 |
| **16** | 5.5 | 13.3 |
| **17** | 4.3 | 39 |
| **18** | 2.5 | 11.8 |
| **19** | 2.6 | 9.3 |
| **20** | 1.2 | 7.1 |
| **21** | 1.9 | 7 |
| **22** | 1.3 | 10.9 |
| **23** | 1.3 | 9.5 |
| **24** | 2.8 | 13.9 |
| **25** | 1.2 | 4.7 |
| **26** | 18 | 33 |
| **27** | 1.8 | 12.9 |
| **28** | 1.1 | 3.6 |
| **29** | 1.1 | 3.7 |
| **30** | 0.9 | 5.0 |
| **31** | 1.7 | 3.8 |
| **32** | 0.5 | 4.4 |
| **33** | 3.3 | 10.9 |
| **34** | 0.5 | 3.8 |
| **35** | 2.9 | 9 |
| **36** | 7.8 | 28 |
| **37** | 3.2 | 28 |
| **38** | 1.8 | 9 |
| **39** | 2.3 | 11 |
| **40** | 1.7 | 10.5 |
| **41** | 2.9 | 12.5 |
| **42** | 1.7 | 13.7 |
| **43** | 1.1 | 13.5 |
| **44** | 0.4 | 2.8 |
| **45** | 0.2 | 1.8 |
| **46** | 0.4 | 1.1 |
| **47** | 0.5 | 3.5 |
| **48** | 0.5 | 1.6 |
| **49** | 0.8 | 3.1 |
| **50** | 1.6 | 9.3 |
| **51** | 10.7 | 29.8 |
| **52** | 10 | 33 |
| **53** | 4.3 | 12.4 |
| **54** | 1.6 | 5.9 |
| **55** | 7.5 | 14.8 |
| **57** | 1.7 | 5.1 |
| **58** | 1.4 | 3.9 |
| **59** | 1.6 | 3.8 |
| **60** | 3 | 8.1 |
| **61** | 1.2 | 6.7 |
| **62** | 0.7 | 3.2 |
| **63** | 3.2 | 17.8 |
| **64** | 3.5 | 43 |
| **65** | 2.8 | 14 |
| **66** | 24.6 | 113 |
| **67** | < 3.1 | 17.5 |
| **68** | 0.7 | 8 |
| **69** | < 3.1 | 17 |
| **70** | < 3.1 | 16 |
| **71** | 18 | 62 |
| **72** | 10 | 91 |
| **73** | 3.3 | 26 |
| **Chloroquine** | 4.2 | 3.8 |
| **Artesunate** | 2.7 | 1 |

### In vivo antimalarial efficacy studies

*In vivo* antimalarial activity is assessed for groups of three female NMRI mice (20-22 g) intravenously infected on day 0 with P. berghei strain GFP-ANKA (0.2 mL heparinized saline suspension containing 2 x 10⁷ parasitized erythrocytes). In control mice, parasitemia typically rises to approximately 40% by day 3 after infection. Compounds are formulated in Tween 80/ethanol (7%/3%) usually at concentrations of 10 mg/mL. Compounds are administered in a volume of 10 mL/kg orally as single doses (1x100 mg/kg, 24 h after infection). 48 h after drug treatment (day 3 post-infection), 1 µl tail blood is taken, resuspended in 1 mL PBS buffer and parasitemia determined with a FACScan (Becton Dickinson) by counting 100'000 red blood cells. Activity is calculated as the difference between the mean value of the control group and treated groups expressed as a percent relative to the control group.

**Table 2: Activity (inhibition of parasitemia) of compounds of formula I after single oral dose of 100 mg/kg expressed as a percent relative to the control group**

| **Compound of Example #** | **Activity [%]** | **Compound of Example #** | **Activity [%]** |
|---|---|---|---|
| 1* | < 40 | 34 | 99.2 |
| 2 | 94.1 | 35 | 98.7 |
| 3 | 98.7 | 36 | 46.8 |
| 4 | 97.4 | 38 | 98.5 |
| 5 | < 40 | 39 | 99 |
| 6 | 80.4 | 40 | 93.6 |
| 7 | 44.7 | 41 | 71 |
| 8 | 99.4 | 42 | 97.3 |
| 9 | 99.1 | 43 | 98.7 |
| 10 | 96 | 44 | 99.0 |
| 15 | 99 | 45 | 99.4 |
| 16 | 85.9 | 46 | 98.9 |
| 18 | 99.1 | 47 | 98.4 |
| 19 | 99.2 | 48 | 99 |
| 20 | 98.5 | 49 | 98.6 |
| 21 | 98.8 | 50 | 98 |
| 22 | 99.3 | 52 | < 40 |
| 23 | 99.1 | 53 | 52.3 |
| 24 | 79.2 | 54 | 93.7 |
| 25 | 99.1 | 55 | < 40 |
| 27 | < 40 | 57 | 98.2 |
| 28 | 99.3 | 58 | 98.2 |
| 29 | 99.2 | 59 | 96.5 |
| 30 | 97.7 | 60 | 78.3 |
| 31 | 88.9 | 61 | 98.8 |
| 32 | 98.5 | 62 | 99.4 |
| 33 | 97.4 | | |

| | | | |
|---|---|---|---|
| * Reference Example | | | |

## Claims

1. A compound of the formula I: 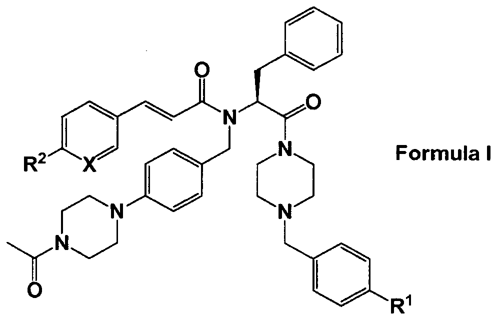 wherein
◆ **X** is CH or N;
**R¹** represents -NO₂, -N(CH₃)₂, or -NCH₃(CH₂CH₂OH); and
**R²** represents hydrogen, methyl, ethyl, n-propyl, isopropyl, tert-butyl, cyano, halogen, methoxy, ethoxy, n-propoxy, isopropoxy, trifluoromethyl, difluoromethoxy, methylsulfonyl, acetyl, or acetylamino; or
◆ **X** is CH, **R¹** is hydrogen, and **R²** is ethyl, isopropyl, tert-butyl, ethoxy, n-propoxy, isopropoxy, methylsulfonyl, acetylamino, or methoxycarbonyl; or
◆ **X** is CH, **R¹** is cyano, and **R²** is ethyl, isopropyl, tert-butyl, ethoxy, n-propoxy, isopropoxy, trifluoromethyl, difluoromethoxy, trifluoromethoxy, methylsulfonyl, or acetylamino; or
◆ **X** is CH, **R¹** is chloro, and **R²** is ethyl, isopropyl, tert-butyl, ethoxy, n-propoxy, isopropoxy, difluoromethoxy, methylsulfonyl, or acetylamino; or
◆ **X** is CH, **R¹** is methoxy or isopropoxy, and **R²** is trifluoromethyl; or
◆ **X** is CH, **R¹** is methylsulfonyl or ethylsulfonyl, and **R²** is trifluoromethyl, ethyl, isopropyl, tert-butyl, ethoxy, n-propoxy, isopropoxy, or difluoromethoxy;
or a salt of such a compound.

2. A compound according to claim 1, wherein
◆ **X** is CH or N;
**R¹** represents -NO₂, -N(CH₃)₂, or -NCH₃(CH₂CH₂OH); and
**R²** represents hydrogen, methyl, ethyl, n-propyl, isopropyl, tert-butyl, cyano, halogen, methoxy, ethoxy, n-propoxy, isopropoxy, trifluoromethyl, difluoromethoxy, methylsulfonyl, acetyl, or acetylamino; or
◆ **X** is CH, **R¹** is hydrogen, and **R²** is ethyl, isopropyl, tert-butyl, ethoxy, n-propoxy, isopropoxy, methylsulfonyl, acetylamino, or methoxycarbonyl; or
◆ **X** is CH, **R¹** is cyano, and **R²** is ethyl, isopropyl, tert-butyl, ethoxy, n-propoxy, isopropoxy, trifluoromethyl, difluoromethoxy, trifluoromethoxy, methylsulfonyl, or acetylamino; or
◆ **X** is CH, **R¹** is chloro, and **R²** is ethyl, isopropyl, tert-butyl, ethoxy, n-propoxy, isopropoxy, difluoromethoxy, methylsulfonyl, or acetylamino; or
◆ **X** is CH, **R¹** is methoxy or isopropoxy, and **R²** is trifluoromethyl;
or a salt of such a compound.

3. A compound according to claim 1, wherein
◆ **X** is CH or N;
**R¹** represents -NO₂, -N(CH₃)₂, or -NCH₃(CH₂CH₂OH); and
**R²** represents ethyl, isopropyl, tert-butyl, methoxy, ethoxy, n-propoxy, isopropoxy, trifluoromethyl, difluoromethoxy, methylsulfonyl, or acetylamino; or
◆ **X** is CH, **R¹** is hydrogen, and **R²** is ethyl, isopropyl, tert-butyl, ethoxy, n-propoxy, isopropoxy, methylsulfonyl, acetylamino, or methoxycarbonyl; or
◆ **X** is CH, **R¹** is cyano, and **R²** is ethyl, isopropyl, tert-butyl, ethoxy, n-propoxy, isopropoxy, trifluoromethyl, difluoromethoxy, trifluoromethoxy, methylsulfonyl, or acetylamino; or
◆ **X** is CH, **R¹** is chloro, and **R²** is ethyl, isopropyl, tert-butyl, ethoxy, n-propoxy, isopropoxy, difluoromethoxy, methylsulfonyl, or acetylamino; or
◆ **X** is CH, **R¹** is methoxy or isopropoxy, and **R²** is trifluoromethyl;
or a salt of such a compound.

4. A compound according to claim 1, wherein
**R¹** represents -NO₂, -N(CH₃)₂, or -NCH₃(CH₂CH₂OH); and
**R²** represents hydrogen, methyl, ethyl, n-propyl, isopropyl, tert-butyl, cyano, halogen, methoxy, ethoxy, n-propoxy, isopropoxy, trifluoromethyl, difluoromethoxy, methylsulfonyl, acetyl, or acetylamino;
or a salt of such a compound.

5. A compound according to claim 1, wherein
**R¹** represents -NO₂, -N(CH₃)₂, or -NCH₃(CH₂CH₂OH); and
**R²** represents ethyl, isopropyl, tert-butyl, methoxy, ethoxy, n-propoxy, isopropoxy, trifluoromethyl, difluoromethoxy, methylsulfonyl, or acetylamino;
or a salt of such a compound.

6. A compound according to claim 4 or 5, wherein **X** is CH, or a salt of such a compound.

7. A compound according to claim 4 or 5, wherein **X** is N, or a salt of such a compound.

8. A compound according to any one of claims 4 to 7, wherein **R¹** represents -NO₂, or a salt of such a compound.

9. A compound according to any one of claims 4 to 7, wherein **R¹** represents -N(CH₃)₂, or a salt of such a compound.

10. A compound according to any one of claims 4 to 7, wherein **R¹** represents -NCH₃(CH₂CH₂OH), or a salt of such a compound.

11. A compound according to any one of claims 4 to 10, wherein **R²** is ethyl, isopropyl, tert-butyl, ethoxy, n-propoxy, or isopropoxy, or a salt of such a compound.

12. A compound according to claim 11, wherein **R²** is isopropoxy, or a salt of such a compound.

13. A compound according to any one of claims 4 to 10, wherein **R²** is trifluoromethyl, difluoromethoxy, methylsulfonyl, or acetylamino, or a salt of such a compound.

14. A compound according to any one of claims 4 to 10, wherein **R²** is methoxy, or a salt of such a compound.

15. A compound according to any one of claims 4 to 10, wherein **R²** is hydrogen, methyl, n-propyl, cyano, halogen, or acetyl, or a salt of such a compound.

16. A compound according to claim 1, selected from the group consisting of:
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-trifluoromethyl-phenyl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(6-trifluoromethyl-pyridin-3-yl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(6-methoxy-pyridin-3-yl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(6-ethoxy-pyridin-3-yl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-ethoxy-phenyl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-methanesulfonyl-phenyl)-acrylamide,
(S)-3-(4-Acetylamino-phenyl)-N-[4-(4-acetyl-piperazin-1-yl)-benzyl]-N-{1-benzyl-2-[4-(4-dimethylamino-benzyl)-piperazin-1-yl]-2-oxo-ethyl}-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-propoxy-phenyl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-isopropoxy-phenyl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-ethyl-phenyl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-difluoromethoxy-phenyl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-tert-butyl-phenyl)-acrylamide,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-isopropyl-phenyl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-trifluoromethyl-phenyl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(6-trifluoromethyl-pyridin-3-yl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(6-methoxy-pyridin-3-yl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(6-ethoxy-pyridin-3-yl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-ethoxy-phenyl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-methanesulfonyl-phenyl)-acrylamide,
(S)-3-(4-Acetylamino-phenyl)-N-[4-(4-acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-propoxy-phenyl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-isopropoxy-phenyl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-ethyl-phenyl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-difluoromethoxy-phenyl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-isopropyl-phenyl)-acrylamide,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-tert-butyl-phenyl)-acrylamide, and
(S)-N-[4-(4-Acetyl-piperazin-1-yl)-benzyl]-N-{1-benzyl-2-[4-(4-nitro-benzyl)-piperazin-1-yl]-2-oxo-ethyl}-3-(4-trifluoromethyl-phenyl)-acrylamide,
or salts of these compounds.

17. A compound according to claim 1, selected from the group consisting of:
(S)-N-[4-(4-Acetyl-piperazin-1-yl)benzyl]-N-{1-(4-(4-(dimethylamino)benzyl)piperazin-1-yl)-1-oxo-3-phenylpropan-2-yl}cinnamamide,
(S)-N-[4-(4-Acetyl-piperazin-1-yl)benzyl]-N-{1-[4-(4-(dimethylamino)benzyl)piperazin-1-yl]-1-oxo-3-phenylpropan-2-yl}-3-(p-tolyl)acrylamide,
(S)-N-[4-(4-Acetyl-piperazin-1-yl)benzyl]-N-{1-(4-(4-(dimethylamino)benzyl)piperazin-1-yl)-1-oxo-3-phenylpropan-2-yl}-3-(4-propylphenyl)acrylamide,
(S)-N-[4-(4-Acetyl-piperazin-1-yl)benzyl]-N-{1-(4-(4-(dimethylamino)benzyl)piperazin-1-yl)-1-oxo-3-phenylpropan-2-yl}-3-(4-methoxyphenyl)acrylamide,
(S)-3-(4-Acetyl-phenyl)-N-[4-(4-acetyl-piperazin-1-yl)benzyl]-N-{1-(4-(4-(dimethylamino)benzyl)piperazin-1-yl)-1-oxo-3-phenylpropan-2-yl}acrylamide,
(S)-N-[4-(4-Acetyl-piperazin-1-yl)benzyl]-3-(4-cyanophenyl)-N-{1-(4-(4-(dimethylamino)benzyl)piperazin-1-yl)-1-oxo-3-phenylpropan-2-yl}acrylamide,
(S)-N-[4-(4-Acetyl-piperazin-1-yl)benzyl]-N-{1-(4-(4-(dimethylamino)benzyl)piperazin-1-yl)-1-oxo-3-phenylpropan-2-yl}-3-(4-fluorophenyl)acrylamide, and
(S)-N-[4-(4-Acetyl-piperazin-1-yl)benzyl]-3-(4-chlorophenyl)-N-{1-(4-(4-(dimethylamino)benzyl)piperazin-1-yl)-1-oxo-3-phenylpropan-2-yl}acrylamide,
or salts of these compounds.

18. A compound according to claim 1, selected from the group consisting of:
(S)-N-[4-(4-Acetyl-piperazin-1-yl)-benzyl]-N-[1-benzyl-2-(4-benzyl-piperazin-1-yl)-2-oxo-ethyl]-3-(4-ethyl-phenyl)-acrylamide,
(S)-N-[4-(4-Acetyl-piperazin-1-yl)-benzyl]-N-[1-benzyl-2-(4-benzyl-piperazin-1-yl)-2-oxo-ethyl]-3-(4-tert-butyl-phenyl)-acrylamide,
(S)-N-[4-(4-Acetyl-piperazin-1-yl)-benzyl]-N-[1-benzyl-2-(4-benzyl-piperazin-1-yl)-2-oxoethyl]-3-(4-ethoxy-phenyl)-acrylamide,
(S)-4-(2-{[4-(4-Acetyl-piperazin-1-yl)-benzyl]-[1-benzyl-2-(4-benzyl-piperazin-1-yl)-2-oxo-ethyl]-carbamoyl}-vinyl)-benzoic acid methyl ester,
(S)-N-[4-(4-Acetyl-piperazin-1-yl)-benzyl]-N-[1-benzyl-2-(4-benzyl-piperazin-1-yl)-2-oxo-ethyl]-3-(4-methanesulfonyl-phenyl)-acrylamide,
(S)-3-(4-Acetylamino-phenyl)-N-[4-(4-acetyl-piperazin-1-yl)-benzyl]-N-[1-benzyl-2-(4-benzyl-piperazin-1-yl)-2-oxo-ethyl]-acrylamide,
(S)-N-[4-(4-Acetyl-piperazin-1-yl)-benzyl]-N-[1-benzyl-2-(4-benzyl-piperazin-1-yl)-2-oxo-ethyl]-3-(4-propoxy-phenyl)-acrylamide,
(S)-N-[4-(4-Acetyl-piperazin-1-yl)-benzyl]-N-[1-benzyl-2-(4-benzyl-piperazin-1-yl)-2-oxo-ethyl]-3-(4-isopropoxy-phenyl)-acrylamide,
(S)-N-[4-(4-Acetyl-piperazin-1-yl)-benzyl]-N-[1-benzyl-2-(4-benzyl-piperazin-1-yl)-2-oxo-ethyl]-3-(4-isopropyl-phenyl)-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-trifluoromethyl-phenyl)-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-ethoxy-phenyl)-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-methanesulfonyl-phenyl)-acrylamide,
(S)-3-(4-Acetylamino-phenyl)-N-[4-(4-acetyl-piperazin-1-yl)-benzyl]-N-{1-benzyl-2-[4-(4-cyano-benzyl)-piperazin-1-yl]-2-oxo-ethyl}-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-propoxy-phenyl)-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-isopropoxy-phenyl)-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-ethyl-phenyl)-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-difluoromethoxy-phenyl)-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-isopropyl-phenyl)-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-trifluoromethoxy-phenyl)-acrylamide,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-tert-butyl-phenyl)-acrylamide,
(S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-ethoxy-phenyl)-acrylamide,
(S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-methanesulfonyl-phenyl)-acrylamide,
(S)-3-(4-Acetylamino-phenyl)-N-[4-(4-acetyl-piperazin-1-yl)-benzyl]-N-{1-benzyl-2-[4-(4-chloro-benzyl)-piperazin-1-yl]-2-oxo-ethyl}-acrylamide,
(S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-propoxy-phenyl)-acrylamide,
(S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-isopropoxy-phenyl)-acrylamide,
(S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-ethyl-phenyl)-acrylamide,
(S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-difluoromethoxy-phenyl)-acrylamide,
(S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-isopropyl-phenyl)-acrylamide,
(S)-N-[4-(4-Acetyl-piperazin-1-yl)-benzyl]-N-{1-benzyl-2-[4-(4-chloro-benzyl)-piperazin-1-yl]-2-oxo-ethyl}-3-(4-tert-butyl-phenyl)-acrylamide,
(S)-N-[4-(4-Acetyl-piperazin-1-yl)-benzyl]-N-{1-benzyl-2-[4-(4-methoxy-benzyl)-piperazin-1-yl]-2-oxo-ethyl}-3-(4-trifluoromethyl-phenyl)-acrylamide, and
(S)-N-[4-(4-Acetyl-piperazin-1-yl)-benzyl]-N-{1-benzyl-2-[4-(4-isopropoxy-benzyl)-piperazin-1-yl]-2-oxo-ethyl}-3-(4-trifluoromethyl-phenyl)-acrylamide,
or salts of these compounds.

19. A compound according to claim 1, selected from the group consisting of:
(S)-N-[4-(4-Acetyl-piperazin-1-yl)-benzyl]-N-{1-benzyl-2-[4-(4-(methylsulfonyl)-benzyl)-piperazin-1-yl]-2-oxo-ethyl}-3-(4-isopropoxyphenyl)-acrylamide,
(S)-N-[4-(4-Acetyl-piperazin-1-yl)-benzyl]-N-{1-benzyl-2-[4-(4-(methylsulfonyl)-benzyl)-piperazin-1-yl]-2-oxo-ethyl}-3-(4-tert-butyl-phenyl)-acrylamide,
(S)-N-[4-(4-Acetyl-piperazin-1-yl)-benzyl]-N-{1-benzyl-2-[4-(4-(methylsulfonyl)-benzyl)-piperazin-1-yl]-2-oxo-ethyl}-3-(4-propoxyphenyl)-acrylamide,
(S)-N-(4-(4-acetylpiperazin-1-yl)benzyl)-N-(1-(4-(4-(methylsulfonyl)benzyl)piperazin-1-yl)-1-oxo-3-phenylpropan-2-yl)-3-(4-(trifluoromethyl)phenyl)acrylamide,
(S)-N-[4-(4-Acetyl-piperazin-1-yl)-benzyl]-N-{1-benzyl-2-[4-(4-(ethylsulfonyl)-benzyl)-piperazin-1-yl]-2-oxo-ethyl}-3-(4-propoxyphenyl)-acrylamide, and
(S)-N-(4-(4-acetylpiperazin-1-yl)benzyl)-N-(1-(4-(4-(ethylsulfonyl)benzyl)piperazin-1-yl)-1-oxo-3-phenylpropan-2-yl)-3-(4-(trifluoromethyl)phenyl)acrylamide,
or salts of these compounds.

20. A pharmaceutical composition comprising a compound according to any one of claims 1 to 19, or a pharmaceutically acceptable salt thereof, and a pharmaceutically acceptable carrier material.

21. A compound according to any one of claims 1 to 19, or a pharmaceutically acceptable salt thereof, or a composition according to claim 20, for use as a medicament.

22. A compound according to any one of claims 1 to 19, or a pharmaceutically acceptable salt thereof, for use in the treatment and/or prevention of protozoal infections.

23. A compound according to any one of claims 1 to 19, or a pharmaceutically acceptable salt thereof, for use in the treatment and/or prevention of malaria.

24. Use of a compound according to any one of claims 1 to 19, or a pharmaceutically acceptable salt thereof, for the preparation of a pharmaceutical composition for the treatment and/or prevention of protozoal infections.

25. The use according to claim 24 for the treatment and/or prevention of malaria.

## Patentansprüche

1. Verbindung der **Formel I:** 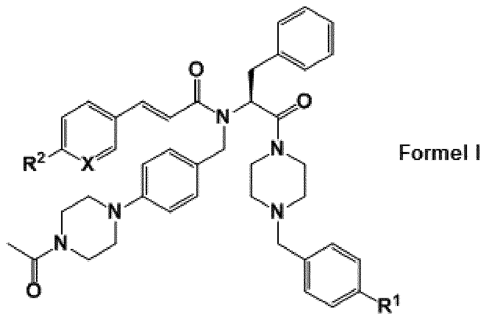 wobei
◆ **X** CH oder N ist;
**R¹** -NO₂, -N(CH₃)₂ oder -NCH₃(CH₂CH₂OH) repräsentiert; und
**R²** Wasserstoff, Methyl, Ethyl, n-Propyl, Isopropyl, tert-Butyl, Cyano, Halogen, Methoxy, Ethoxy, n-Propoxy, Isopropoxy, Trifluormethyl, Difluormethoxy, Methylsulfonyl, Acetyl oder Acetylamino repräsentiert; oder
◆ **X** CH ist, **R¹** Wasserstoff ist und **R²** Ethyl, Isopropyl, tert-Butyl, Ethoxy, n-Propoxy, Isopropoxy, Methylsulfonyl, Acetylamino oder Methoxycarbonyl ist; oder
◆ **X** CH ist, **R¹** Cyano ist und **R²** Ethyl, Isopropyl, tert-Butyl, Ethoxy, n-Propoxy, Isopropoxy, Trifluormethyl, Difluormethoxy, Trifluormethoxy, Methylsulfonyl oder Acetylamino ist; oder
◆ **X** CH ist, **R¹** Chlor ist und **R²** Ethyl, Isopropyl, tert-Butyl, Ethoxy, n-Propoxy, Isopropoxy, Difluormethoxy, Methylsulfonyl oder Acetylamino ist; oder
◆ **X** CH ist, **R¹** Methoxy oder Isopropoxy ist und **R²** Trifluormethyl ist; oder
◆ **X** CH ist, **R¹** Methylsulfonyl oder Ethylsulfonyl ist und **R²** Trifluormethyl, Ethyl, Isopropyl, tert-Butyl, Ethoxy, n-Propoxy, Isopropoxy oder Difluormethoxy ist;
oder ein Salz einer solchen Verbindung.

2. Verbindung nach Anspruch 1, wobei
◆ **X** CH oder N ist;
**R¹** -NO₂, -N(CH₃)₂ oder -NCH₃(CH₂CH₂OH) repräsentiert; und
**R²** Wasserstoff, Methyl, Ethyl, n-Propyl, Isopropyl, tert-Butyl, Cyano, Halogen, Methoxy, Ethoxy, n-Propoxy, Isopropoxy, Trifluormethyl, Difluormethoxy, Methylsulfonyl, Acetyl oder Acetylamino repräsentiert; oder
◆ **X** CH ist, **R¹** Wasserstoff ist und **R²** Ethyl, Isopropyl, tert-Butyl, Ethoxy, n-Propoxy, Isopropoxy, Methylsulfonyl, Acetylamino oder Methoxycarbonyl ist; oder
◆ **X** CH ist, **R¹** Cyano ist und **R²** Ethyl, Isopropyl, tert-Butyl, Ethoxy, n-Propoxy, Isopropoxy, Trifluormethyl, Difluormethoxy, Trifluormethoxy, Methylsulfonyl oder Acetylamino ist; oder
◆ **X** CH ist, **R¹** Chlor ist und **R²** Ethyl, Isopropyl, tert-Butyl, Ethoxy, n-Propoxy, Isopropoxy, Difluormethoxy, Methylsulfonyl oder Acetylamino ist; oder
◆ **X** CH ist, **R¹** Methoxy oder Isopropoxy ist und **R²** Trifluormethyl ist;
oder ein Salz einer solchen Verbindung.

3. Verbindung nach Anspruch 1, wobei
◆ **X** CH oder N ist;
**R¹** -NO₂, -N(CH₃)₂ oder -NCH₃(CH₂CH₂OH) repräsentiert; und
**R²** Ethyl, Isopropyl, tert-Butyl, Methoxy, Ethoxy, n-Propoxy, Isopropoxy, Trifluormethyl, Difluormethoxy, Methylsulfonyl oder Acetylamino repräsentiert; oder
◆ **X** CH ist, **R¹** Wasserstoff ist und **R²** Ethyl, Isopropyl, tert-Butyl, Ethoxy, n-Propoxy, Isopropoxy, Methylsulfonyl, Acetylamino oder Methoxycarbonyl ist; oder
◆ **X** CH ist, **R¹** Cyano ist und **R²** Ethyl, Isopropyl, tert-Butyl, Ethoxy, n-Propoxy, Isopropoxy, Trifluormethyl, Difluormethoxy, Trifluormethoxy, Methylsulfonyl oder Acetylamino ist; oder
◆ **X** CH ist, **R¹** Chlor ist und **R²** Ethyl, Isopropyl, tert-Butyl, Ethoxy, n-Propoxy, Isopropoxy, Difluormethoxy, Methylsulfonyl oder Acetylamino ist; oder
◆ **X** CH ist, **R¹** Methoxy oder Isopropoxy ist und **R²** Trifluormethyl ist;
oder ein Salz einer solchen Verbindung.

4. Verbindung nach Anspruch 1, wobei
**R¹** -NO₂, -N(CH₃)₂ oder -NCH₃(CH₂CH₂OH) repräsentiert; und
**R²** Wasserstoff, Methyl, Ethyl, n-Propyl, Isopropyl, tert-Butyl, Cyano, Halogen, Methoxy, Ethoxy, n-Propoxy, Isopropoxy, Trifluormethyl, Difluormethoxy, Methylsulfonyl, Acetyl oder Acetylamino repräsentiert;
oder ein Salz einer solchen Verbindung.

5. Verbindung nach Anspruch 1, wobei
**R¹** -NO₂, -N(CH₃)₂ oder -NCH₃(CH₂CH₂OH) repräsentiert; und
**R²** Ethyl, Isopropyl, tert-Butyl, Methoxy, Ethoxy, n-Propoxy, Isopropoxy, Trifluormethyl, Difluormethoxy, Methylsulfonyl oder Acetylamino repräsentiert;
oder ein Salz einer solchen Verbindung.

6. Verbindung nach Anspruch 4 oder 5, wobei **X** CH ist, oder ein Salz einer solchen Verbindung.

7. Verbindung nach Anspruch 4 oder 5, wobei **X** N ist, oder ein Salz einer solchen Verbindung.

8. Verbindung nach einem der Ansprüche 4 bis 7, wobei **R¹** -NO₂ repräsentiert, oder ein Salz einer solchen Verbindung.

9. Verbindung nach einem der Ansprüche 4 bis 7, wobei **R¹** -N(CH₃)₂ repräsentiert, oder ein Salz einer solchen Verbindung.

10. Verbindung nach einem der Ansprüche 4 bis 7, wobei **R¹** -NCH₃(CH₂CH₂OH) repräsentiert, oder ein Salz einer solchen Verbindung.

11. Verbindung nach einem der Ansprüche 4 bis 10, wobei **R²** Ethyl, Isopropyl, tert-Butyl, Ethoxy, n-Propoxy oder Isopropoxy ist, oder ein Salz einer solchen Verbindung.

12. Verbindung nach Anspruch 11, wobei **R²** Isopropoxy ist, oder ein Salz einer solchen Verbindung.

13. Verbindung nach einem der Ansprüche 4 bis 10, wobei **R²** Trifluormethyl, Difluormethoxy, Methylsulfonyl oder Acetylamino ist, oder ein Salz einer solchen Verbindung.

14. Verbindung nach einem der Ansprüche 4 bis 10, wobei **R²** Methoxy ist, oder ein Salz einer solchen Verbindung.

15. Verbindung nach einem der Ansprüche 4 bis 10, wobei **R²** Wasserstoff, Methyl, n-Propyl, Cyano, Halogen oder Acetyl ist, oder ein Salz einer solchen Verbindung.

16. Verbindung nach Anspruch 1, die ausgewählt ist aus der Gruppe bestehend aus:
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-trifluormethyl-phenyl)-acrylamid,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(6-trifluormethyl-pyridin-3-yl)-acrylamid,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(6-methoxy-pyridin-3-yl)-acrylamid,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(6-ethoxy-pyridin-3-yl)-acrylamid,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-ethoxy-phenyl)-acrylamid,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-methansulfonyl-phenyl)-acrylamid,
(S)-3-(4-Acetylamino-phenyl)-N-[4-(4-acetyl-piperazin-1-yl)-benzyl]-N-{1-benzyl-2-[4-(4-dimethylamino-benzyl)-piperazin-1-yl]-2-oxo-ethyl}-acrylamid,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-propoxy-phenyl)-acrylamid,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-isopropoxy-phenyl)-acrylamid,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-ethyl-phenyl)-acrylamid,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-difluormethoxy-phenyl)-acrylamid,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-tert-butyl-phenyl)-acrylamid,
(S)-N-[4-(4-Acetyl-piperazin-1-yl)-benzyl]-N-{1-benzyl-2-[4-(4-dimethylamino-benzyl)-piperazin-1-yl]-2-oxo-ethyl}-3-(4-isopropyl-phenyl)-acrylamid,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-trifluormethyl-phenyl)-acrylamid,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(6-trifluormethyl-pyridin-3-yl)-acrylamid,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(6-methoxy-pyridin-3-yl)-acrylamid,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(6-ethoxy-pyridin-3-yl)-acrylamid,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-ethoxy-phenyl)-acrylamid,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-methansulfonyl-phenyl)-acrylamid,
(S)-3-(4-Acetylamino-phenyl)-N-[4-(4-acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-acrylamid,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-propoxy-phenyl)-acrylamid,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-isopropoxy-phenyl)-acrylamid,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-ethyl-phenyl)-acrylamid,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-difluormethoxy-phenyl)-acrylamid,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-isopropyl-phenyl)-acrylamid,
(S)-N-[4-(4-Acetyl-piperazin-1-yl)-benzyl]-N-[1-benzyl-2-(4-{4-[(2-hydroxy-ethyl)-methyl-amino]-benzyl}-piperazin-1-yl)-2-oxo-ethyl]-3-(4-tert-butyl-phenyl)-acrylamid und
(S)-N-[4-(4-Acetyl-piperazin-1-yl)-benzyl]-N-{1-benzyl-2-[4-(4-nitro-benzyl)-piperazin-1-yl]-2-oxo-ethyl}-3-(4-trifluormethyl-phenyl)-acrylamid,
oder Salze dieser Verbindungen.

17. Verbindung nach Anspruch 1, die ausgewählt ist aus der Gruppe bestehend aus:
(S)-N-[4-(4-Acetyl-piperazin-1-yl)benzyl]-N-{1-(4-(4-(dimethylamino)benzyl)piperazin-1-yl)-1-oxo-3-phenylpropan-2-yl}cinnamamid,
(S)-N-[4-(4-Acetyl-piperazin-1-yl)benzyl]-N-{1-[4-(4-(dimethylamino)benzyl)piperazin-1-yl]-1-oxo-3-phenylpropan-2-yl}-3-(p-tolyl)acrylamid,
(S)-N-[4-(4-Acetyl-piperazin-1-yl)benzyl]-N-{1-(4-(4-(dimethylamino)benzyl)piperazin-1-yl)-1-oxo-3-phenylpropan-2-yl}-3-(4-propylphenyl)acrylamid,
(S)-N-[4-(4-Acetyl-piperazin-1-yl)benzyl]-N-{1-(4-(4-(dimethylamino)benzyl)piperazin-1-yl)-1-oxo-3-phenylpropan-2-yl}-3-(4-methoxyphenyl)acrylamid,
(S)-3-(4-Acetyl-phenyl)-N-[4-(4-acetyl-piperazin-1-yl)benzyl]-N-{1-(4-(4-(dimethylamino)benzyl)piperazin-1-yl)-1-oxo-3-phenylpropan-2-yl}acrylamid,
(S)-N-[4-(4-Acetyl-piperazin-1-yl)benzyl]-3-(4-cyanophenyl)-N-{1-(4-(4-(dimethylamino)benzyl)piperazin-1-yl)-1-oxo-3-phenylpropan-2-yl}acrylamid,
(S)-N-[4-(4-Acetyl-piperazin-1-yl)benzyl]-N-{1-(4-(4-(dimethylamino)benzyl)piperazin-1-yl)-1-oxo-3-phenylpropan-2-yl}-3-(4-fluorphenyl)acrylamid und
(S)-N-[4-(4-Acetyl-piperazin-1-yl)benzyl]-3-(4-chlorphenyl)-N-{1-(4-(4-(dimethylamino)benzyl)piperazin-1-yl)-1-oxo-3-phenylpropan-2-yl}acrylamid,
oder Salze dieser Verbindungen.

18. Verbindung nach Anspruch 1, die ausgewählt ist aus der Gruppe bestehend aus:
(S)-N-[4-(4-Acetyl-piperazin-1-yl)-benzyl]-N-[1-benzyl-2-(4-benzyl-piperazin-1-yl)-2-oxo-ethyl]-3-(4-ethyl-phenyl)-acrylamid,
(S)-N-[4-(4-Acetyl-piperazin-1-yl)-benzyl]-N-[1-benzyl-2-(4-benzyl-piperazin-1-yl)-2-oxo-ethyl]-3-(4-tert-butyl-phenyl)-acrylamid,
(S)-N-[4-(4-Acetyl-piperazin-1-yl)-benzyl]-N-[1-benzyl-2-(4-benzyl-piperazin-1-yl)-2-oxo-ethyl]-3-(4-ethoxy-phenyl)-acrylamid,
(S)-4-(2-{[4-(4-Acetyl-piperazin-1-yl)-benzyl]-[1-benzyl-2-(4-benzyl-piperazin-1-yl)-2-oxo-ethyl]-carbamoyl}-vinyl)-benzoesäuremethylester,
(S)-N-[4-(4-Acetyl-piperazin-1-yl)-benzyl]-N-[1-benzyl-2-(4-benzyl-piperazin-1-yl)-2-oxo-ethyl]-3-(4-methansulfonyl-phenyl)-acrylamid,
(S)-3-(4-Acetylamino-phenyl)-N-[4-(4-acetyl-piperazin-1-yl)-benzyl]-N-[1-benzyl-2-(4-benzyl-piperazin-1-yl)-2-oxo-ethyl]-acrylamid,
(S)-N-[4-(4-Acetyl-piperazin-1-yl)-benzyl]-N-[1-benzyl-2-(4-benzyl-piperazin-1-yl)-2-oxo-ethyl]-3-(4-propoxy-phenyl)-acrylamid,
(S)-N-[4-(4-Acetyl-piperazin-1-yl)-benzyl]-N-[1-benzyl-2-(4-benzyl-piperazin-1-yl)-2-oxo-ethyl]-3-(4-isopropoxy-phenyl)-acrylamid,
(S)-N-[4-(4-Acetyl-piperazin-1-yl)-benzyl]-N-[1-benzyl-2-(4-benzyl-piperazin-1-yl)-2-oxo-ethyl]-3-(4-isopropyl-phenyl)-acrylamid,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-trifluormethyl-phenyl)-acrylamid,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-ethoxy-phenyl)-acrylamid,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-methansulfonyl-phenyl)-acrylamid,
(S)-3-(4-Acetylamino-phenyl)-N-[4-(4-acetyl-piperazin-1-yl)-benzyl]-N-{1-benzyl-2-[4-(4-cyano-benzyl)-piperazin-1-yl]-2-oxo-ethyl}-acrylamid,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-propoxy-phenyl)-acrylamid,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-isopropoxy-phenyl)-acrylamid,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-ethyl-phenyl)-acrylamid,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-difluormethoxy-phenyl)-acrylamid,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-isopropyl-phenyl)-acrylamid,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-trifluormethoxy-phenyl)-acrylamid,
(S)-N-[4-(4-Acetyl-piperazin-1-yl)-benzyl]-N-{1-benzyl-2-[4-(4-cyano-benzyl)-piperazin-1-yl]-2-oxo-ethyl}-3-(4-tert-butyl-phenyl)-acrylamid,
(S)-N-[4-(4-Acetyl-piperazin-1-yl)-benzyl]-N-{1-benzyl-2-[4-(4-chlor-benzyl)-piperazin-1-yl]-2-oxo-ethyl}-3-(4-ethoxy-phenyl)-acrylamid,
(S)-N-[4-(4-Acetyl-piperazin-1-yl)-benzyl]-N-{1-benzyl-2-[4-(4-chlor-benzyl)-piperazin-1-yl]-2-oxo-ethyl}-3-(4-methansulfonyl-phenyl)-acrylamid,
(S)-3-(4-Acetylamino-phenyl)-N-[4-(4-acetyl-piperazin-1-yl)-benzyl]-N-{1-benzyl-2-[4-(4-chlor-benzyl)-piperazin-1-yl]-2-oxo-ethyl}-acrylamid,
(S)-N-[4-(4-Acetyl-piperazin-1-yl)-benzyl]-N-{1-benzyl-2-[4-(4-chlor-benzyl)-piperazin-1-yl]-2-oxo-ethyl}-3-(4-propoxy-phenyl)-acrylamid,
(S)-N-[4-(4-Acetyl-piperazin-1-yl)-benzyl]-N-{1-benzyl-2-[4-(4-chlor-benzyl)-piperazin-1-yl]-2-oxo-ethyl}-3-(4-isopropoxy-phenyl)-acrylamid,
(S)-N-[4-(4-Acetyl-piperazin-1-yl)-benzyl]-N-{1-benzyl-2-[4-(4-chlor-benzyl)-piperazin-1-yl]-2-oxo-ethyl}-3-(4-ethyl-phenyl)-acrylamid,
(S)-N-[4-(4-Acetyl-piperazin-1-yl)-benzyl]-N-{1-benzyl-2-[4-(4-chlor-benzyl)-piperazin-1-yl]-2-oxo-ethyl}-3-(4-difluormethoxy-phenyl)-acrylamid,
(S)-N-[4-(4-Acetyl-piperazin-1-yl)-benzyl]-N-{1-benzyl-2-[4-(4-chlor-benzyl)-piperazin-1-yl]-2-oxo-ethyl}-3-(4-isopropyl-phenyl)-acrylamid,
(S)-N-[4-(4-Acetyl-piperazin-1-yl)-benzyl]-N-{1-benzyl-2-[4-(4-chlor-benzyl)-piperazin-1-yl]-2-oxo-ethyl}-3-(4-tert-butyl-phenyl)-acrylamid,
(S)-N-[4-(4-Acetyl-piperazin-1-yl)-benzyl]-N-{1-benzyl-2-[4-(4-methoxy-benzyl)-piperazin-1-yl]-2-oxo-ethyl}-3-(4-trifluormethyl-phenyl)-acrylamid und
(S)-N-[4-(4-Acetyl-piperazin-1-yl)-benzyl]-N-{1-benzyl-2-[4-(4-isopropoxy-benzyl)-piperazin-1-yl]-2-oxo-ethyl}-3-(4-trifluormethyl-phenyl)-acrylamid,
oder Salze dieser Verbindungen.

19. Verbindung nach Anspruch 1, die ausgewählt ist aus der Gruppe bestehend aus:
(S)-N-[4-(4-Acetyl-piperazin-1-yl)-benzyl]-N-{1-benzyl-2-[4-(4-(methylsulfonyl)-benzyl)-piperazin-1-yl]-2-oxo-ethyl}-3-(4-isopropoxyphenyl)-acrylamid,
(S)-N-[4-(4-Acetyl-piperazin-1-yl)-benzyl]-N-{1-benzyl-2-[4-(4-(methylsulfonyl)-benzyl)-piperazin-1-yl]-2-oxo-ethyl}-3-(4-tert-butyl-phenyl)-acrylamid,
(S)-N-[4-(4-Acetyl-piperazin-1-yl)-benzyl]-N-{1-benzyl-2-[4-(4-(methylsulfonyl)-benzyl)-piperazin-1-yl]-2-oxo-ethyl}-3-(4-propoxyphenyl)-acrylamid,
(S)-N-(4-(4-Acetylpiperazin-1-yl)benzyl)-N-(1-(4-(4-(methylsulfonyl)benzyl)piperazin-1-yl)-1-oxo-3-phenylpropan-2-yl)-3-(4-(trifluormethyl)phenyl)acrylamid,
(S)-N-[4-(4-Acetyl-piperazin-1-yl)-benzyl]-N-{1-benzyl-2-[4-(4-(ethylsulfonyl)-benzyl)-piperazin-1-yl]-2-oxo-ethyl}-3-(4-propoxyphenyl)-acrylamid und
(S)-N-(4-(4-Acetylpiperazin-1-yl)benzyl)-N-(1-(4-(4-(ethylsulfonyl)benzyl)piperazin-1-yl)-1-oxo-3-phenylpropan-2-yl)-3-(4-(trifluormethyl)phenyl)acrylamid,
oder Salze dieser Verbindungen.

20. Pharmazeutische Zusammensetzung, die eine Verbindung nach einem der Ansprüche 1 bis 19 oder ein pharmazeutisch akzeptables Salz davon und ein pharmazeutisch akzeptables Trägermaterial beinhaltet.

21. Verbindung nach einem der Ansprüche 1 bis 19 oder ein pharmazeutisch akzeptables Salz davon oder eine Zusammensetzung nach Anspruch 20 zur Verwendung als Medikament.

22. Verbindung nach einem der Ansprüche 1 bis 19 oder ein pharmazeutisch akzeptables Salz davon zur Verwendung bei der Behandlung und/oder Verhütung von Protozoeninfektionen.

23. Verbindung nach einem der Ansprüche 1 bis 19 oder ein pharmazeutisch akzeptables Salz davon zur Verwendung bei der Behandlung und/oder Verhütung von Malaria.

24. Verwendung einer Verbindung nach einem der Ansprüche 1 bis 19 oder eines pharmazeutisch akzeptablen Salzes davon zur Herstellung einer pharmazeutischen Zusammensetzung für die Behandlung und/oder Verhütung von Protozoeninfektionen.

25. Verwendung nach Anspruch 24 zur Behandlung und/oder Verhütung von Malaria.

## Revendications

1. Composé de **formule I** : 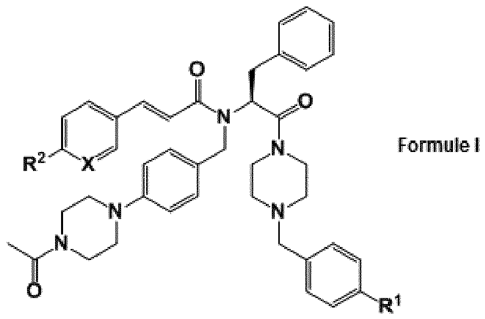 dans lequel
◆ **X** représente CH ou N ;
**R¹** représente -NO₂, -N(CH₃)₂ ou -NCH₃(CH₂CH₂OH) ; et
**R²** représente un hydrogène, méthyle, éthyle, n-propyle, isopropyle, *tert*-butyle, cyano, halogène, méthoxy, éthoxy, n-propoxy, isopropoxy, trifluorométhyle, difluorométhoxy, méthylsulfonyle, acétyle ou acétylamino ; ou
◆ X représente CH, **R¹** représente un hydrogène et **R²** représente un éthyle, isopropyle, *tert*-butyle, éthoxy, n-propoxy, isopropoxy, méthylsulfonyle, acétylamino ou méthoxycarbonyle ; ou
◆ **X** représente CH, **R¹** représente un cyano et **R²** représente un éthyle, isopropyle, *tert*-butyle, éthoxy, n-propoxy, isopropoxy, trifluorométhyle, difluorométhoxy, trifluorométhoxy, méthylsulfonyle ou acétylamino ; ou
◆ **X** représente CH, **R¹** représente un chloro et **R²** représente un éthyle, isopropyle, *tert*-butyle, éthoxy, n-propoxy, isopropoxy, difluorométhoxy, méthylsulfonyle ou acétylamino ; ou
◆ **X** représente CH, **R¹** représente un méthoxy ou un isopropoxy et **R²** représente un trifluorométhyle ; ou
◆ **X** représente CH, **R¹** représente un méthylsulfonyle ou un éthylsulfonyle et **R²** représente un trifluorométhyle, éthyle, isopropyle, *tert*-butyle, éthoxy, n-propoxy, isopropoxy ou difluorométhoxy ;
ou sel de celui-ci.

2. Composé selon la revendication 1 dans lequel
◆ **X** représente CH ou N ;
**R¹** représente -NO₂, -N(CH₃)₂ ou -NCH₃(CH₂CH₂OH) ; et
**R²** représente un hydrogène, méthyle, éthyle, n-propyle, isopropyle, *tert*-butyle, cyano, halogène, méthoxy, éthoxy, n-propoxy, isopropoxy, trifluorométhyle, difluorométhoxy, méthylsulfonyle, acétyle ou acétylamino ; ou
◆ **X** représente CH, **R¹** représente un hydrogène et **R²** représente un éthyle, isopropyle, *tert*-butyle, éthoxy, n-propoxy, isopropoxy, méthylsulfonyle, acétylamino ou méthoxycarbonyle ; ou
◆ **X** représente CH, **R¹** représente un cyano et **R²** représente un éthyle, isopropyle, *tert*-butyle, éthoxy, n-propoxy, isopropoxy, trifluorométhyle, difluorométhoxy, trifluorométhoxy, méthylsulfonyle ou acétylamino ; ou
◆ **X** représente CH, **R¹** représente un chloro et **R²** représente un éthyle, isopropyle, *tert-*butyle, éthoxy, n-propoxy, isopropoxy, difluorométhoxy, méthylsulfonyle ou acétylamino ; ou
◆ **X** représente CH, **R¹** représente un méthoxy ou un isopropoxy et **R²** représente un trifluorométhyle ;
ou sel de celui-ci.

3. Composé selon la revendication 1 dans lequel
◆ **X** représente CH ou N ;
**R¹** représente -NO₂, -N(CH₃)₂ ou -NCH₃(CH₂CH₂OH) ; et
**R²** représente un éthyle, isopropyle, *tert*-butyle, méthoxy, éthoxy, n-propoxy, isopropoxy, trifluorométhyle, difluorométhoxy, méthylsulfonyle ou acétylamino ; ou
◆ **X** représente CH, **R¹** représente un hydrogène et **R²** représente un éthyle, isopropyle, *tert*-butyle, éthoxy, n-propoxy, isopropoxy, méthylsulfonyle, acétylamino ou méthoxycarbonyle ; ou
◆ **X** représente CH, **R¹** représente un cyano et **R²** représente un éthyle, isopropyle, *tert*-butyle, éthoxy, n-propoxy, isopropoxy, trifluorométhyle, difluorométhoxy, trifluorométhoxy, méthylsulfonyle ou acétylamino ; ou
◆ **X** représente CH, **R¹** représente un chloro et **R²** représente un éthyle, isopropyle, *tert*-butyle, éthoxy, n-propoxy, isopropoxy, difluorométhoxy, méthylsulfonyle ou acétylamino ; ou
◆ **X** représente CH, **R¹** représente un méthoxy ou un isopropoxy et **R²** représente un trifluorométhyle ;
ou sel de celui-ci.

4. Composé selon la revendication 1 dans lequel
**R¹** représente -NO₂, -N(CH₃)₂ ou -NCH₃(CH₂CH₂OH) ; et
**R²** représente un hydrogène, méthyle, éthyle, n-propyle, isopropyle, *tert*-butyle, cyano, halogène, méthoxy, éthoxy, n-propoxy, isopropoxy, trifluorométhyle, difluorométhoxy, méthylsulfonyle, acétyle ou acétylamino ;
ou sel de celui-ci.

5. Composé selon la revendication 1 dans lequel
**R¹** représente -NO₂, -N(CH₃)₂ ou -NCH₃(CH₂CH₂OH) ; et
**R²** représente un éthyle, isopropyle, *tert*-butyle, méthoxy, éthoxy, n-propoxy, isopropoxy, trifluorométhyle, difluorométhoxy, méthylsulfonyle ou acétylamino ;
ou sel de celui-ci.

6. Composé selon la revendication 4 ou 5 dans lequel **X** représente CH, ou sel de celui-ci.

7. Composé selon la revendication 4 ou 5 dans lequel **X** représente N, ou sel de celui-ci.

8. Composé selon l'une quelconque des revendications 4 à 7 dans lequel **R¹** représente -NO₂, ou sel de celui-ci.

9. Composé selon l'une quelconque des revendications 4 à 7 dans lequel **R¹** représente -N(CH₃)₂, ou sel de celui-ci.

10. Composé selon l'une quelconque des revendications 4 à 7 dans lequel **R¹** représente -NCH₃(CH₂CH₂OH), ou sel de celui-ci.

11. Composé selon l'une quelconque des revendications 4 à 10 dans lequel **R²** représente un éthyle, isopropyle, *tert*-butyle, éthoxy, n-propoxy ou isopropoxy, ou sel de celui-ci.

12. Composé selon la revendication 11 dans lequel **R²** représente un isopropoxy, ou sel de celui-ci.

13. Composé selon l'une quelconque des revendications 4 à 10 dans lequel **R²** représente un trifluorométhyle, difluorométhoxy, méthylsulfonyle ou acétylamino, ou sel de celui-ci.

14. Composé selon l'une quelconque des revendications 4 à 10 dans lequel **R²** représente un méthoxy, ou sel de celui-ci.

15. Composé selon l'une quelconque des revendications 4 à 10 dans lequel **R²** représente un hydrogène, méthyle, n-propyle, cyano, halogène ou acétyle, ou sel de celui-ci.

16. Composé selon la revendication 1, sélectionné dans le groupe consistant en les suivants :
(S)-N-[4-(4-Acétyl-pipérazin-1-yl)-benzyl]-N-{1-benzyl-2-[4-(4-diméthylamino-benzyl)-pipérazin-1-yl]-2-oxo-éthyl}-3-(4-trifluorométhyl-phényl)-acrylamide,
(S)-N-[4-(4-Acétyl-pipérazin-1-yl)-benzyl]-N-{1-benzyl-2-[4-(4-diméthylamino-benzyl)-pipérazin-1-yl]-2-oxo-éthyl}-3-(6-trifluorométhyl-pyridin-3-yl)-acrylamide,
(S)-N-[4-(4-Acétyl-pipérazin-1-yl)-benzyl]-N-{1-benzyl-2-[4-(4-diméthylamino-benzyl)-pipérazin-1-yl]-2-oxo-éthyl}-3-(6-méthoxy-pyridin-3-yl)-acrylamide,
(S)-N-[4-(4-Acétyl-pipérazin-1-yl)-benzyl]-N-{1-benzyl-2-[4-(4-diméthylamino-benzyl)-pipérazin-1-yl]-2-oxo-éthyl}-3-(6-éthoxy-pyridin-3-yl)-acrylamide,
(S)-N-[4-(4-Acétyl-pipérazin-1-yl)-benzyl]-N-{1-benzyl-2-[4-(4-diméthylamino-benzyl)-pipérazin-1-yl]-2-oxo-éthyl}-3-(4-éthoxy-phényl)-acrylamide,
(S)-N-[4-(4-Acétyl-pipérazin-1-yl)-benzyl]-N-{1-benzyl-2-[4-(4-diméthylamino-benzyl)-pipérazin-1-yl]-2-oxo-éthyl}-3-(4-méthanesulfonyl-phényl)-acrylamide,
(S)-3-(4-Acétylamino-phényl)-N-[4-(4-acétyl-pipérazin-1-yl)-benzyl]-N-{1-benzyl-2-[4-(4-diméthylamino-benzyl)-pipérazin-1-yl]-2-oxo-éthyl}-acrylamide,
(S)-N-[4-(4-Acétyl-pipérazin-1-yl)-benzyl]-N-{1-benzyl-2-[4-(4-diméthylamino-benzyl)-pipérazin-1-yl]-2-oxo-éthyl}-3-(4-propoxy-phényl)-acrylamide,
(S)-N-[4-(4-Acétyl-pipérazin-1-yl)-benzyl]-N-{1-benzyl-2-[4-(4-diméthylamino-benzyl)-pipérazin-1-yl]-2-oxo-éthyl}-3-(4-isopropoxy-phényl)-acrylamide,
(S)-N-[4-(4-Acétyl-pipérazin-1-yl)-benzyl]-N-{1-benzyl-2-[4-(4-diméthylamino-benzyl)-pipérazin-1-yl]-2-oxo-éthyl}-3-(4-éthyl-phényl)-acrylamide,
(S)-N-[4-(4-Acétyl-pipérazin-1-yl)-benzyl]-N-{-benzyl-2-[4-(4-diméthylamino-benzyl)-pipérazin-1-yl]-2-oxo-éthyl}-3-(4-difluorométhoxy-phényl)-acrylamide,
(S)-N-[4-(4-Acétyl-pipérazin-1-yl)-benzyl]-N-{-benzyl-2-[4-(4-diméthylamino-benzyl)-pipérazin-1-yl]-2-oxo-éthyl}-3-(4-*tert*-butyl-phényl)-acrylamide,
(S)-N-[4-(4-Acétyl-pipérazin-1-yl)-benzyl]-N-{-benzyl-2-[4-(4-diméthylamino-benzyl)-pipérazin-1-yl]-2-oxo-éthyl}-3-(4-isopropyl-phényl)-acrylamide,
(S)-N-[4-(4-Acétyl-pipérazin-1-yl)-benzyl]-N-[1-benzyl-2-(4-{-[(2-hydroxy-éthyl)-méthyl-amino]-benzyl}-pipérazin-1-yl)-2-oxo-éthyl]-3-(4-trifluorométhyl-phényl)-acrylamide,
(S)-N-[4-(4-Acétyl-pipérazin-1-yl)-benzyl]-N-[1-benzyl-2-(4-{-[(2-hydroxy-éthyl)-méthyl-amino]-benzyl}-pipérazin-1-yl)-2-oxo-éthyl]-3-(6-trifluorométhyl-pyridin-3-yl)-acrylamide,
(S)-N-[4-(4-Acétyl-pipérazin-1-yl)-benzyl]-N-[1-benzyl-2-(4-{4-[(2-hydroxy-éthyl)-méthyl-amino]-benzyl}-pipérazin-1-yl)-2-oxo-éthyl]-3-(6-méthoxy-pyridin-3-yl)-acrylamide,
(S)-N-[4-(4-Acétyl-pipérazin-1-yl)-benzyl]-N-[1-benzyl-2-(4-{4-[(2-hydroxy-éthyl)-méthyl-amino]-benzyl}-pipérazin-1-yl)-2-oxo-éthyl]-3-(6-éthoxy-pyridin-3-yl)-acrylamide,
(S)-N-[4-(4-Acétyl-pipérazin-1-yl)-benzyl]-N-[1-benzyl-2-(4-{4-[(2-hydroxy-éthyl)-méthyl-amino]-benzyl}-pipérazin-1-yl)-2-oxo-éthyl]-3-(4-éthoxy-phényl)-acrylamide,
(S)-N-[4-(4-Acétyl-pipérazin-1-yl)-benzyl]-N-[1-benzyl-2-(4-{4-[(2-hydroxy-éthyl)-méthyl-amino]-benzyl}-pipérazin-1-yl)-2-oxo-éthyl]-3-(4-méthanesulfonyl-phényl)-acrylamide,
(S)-3-(4-Acétylamino-phényl)-N-[4-(4-acétyl-pipérazin-1-yl)-benzyl]-N-[1-benzyl-2-(4-{4-[(2-hydroxy-éthyl)-méthyl-amino]-benzyl}-pipérazin-1-yl)-2-oxo-éthyl]-acrylamide,
(S)-N-[4-(4-Acétyl-pipérazin-1-yl)-benzyl]-N-[1-benzyl-2-(4-{4-[(2-hydroxy-éthyl)-méthyl-amino]-benzyl}-pipérazin-1-yl)-2-oxo-éthyl]-3-(4-propoxy-phényl)-acrylamide,
(S)-N-[4-(4-Acétyl-pipérazin-1-yl)-benzyl]-N-[1-benzyl-2-(4-{4-[(2-hydroxy-éthyl)-méthyl-amino]-benzyl}-pipérazin-1-yl)-2-oxo-éthyl]-3-(4-isopropoxy-phényl)-acrylamide,
(S)-N-[4-(4-Acétyl-pipérazin-1-yl)-benzyl]-N-[1-benzyl-2-(4-{4-[(2-hydroxy-éthyl)-méthyl-amino]-benzyl}-pipérazin-1-yl)-2-oxo-éthyl]-3-(4-éthyl-phényl)-acrylamide,
(S)-N-[4-(4-Acétyl-pipérazin-1-yl)-benzyl]-N-[1-benzyl-2-(4-{4-[(2-hydroxy-éthyl)-méthyl-amino]-benzyl}-pipérazin-1-yl)-2-oxo-éthyl]-3-(4-difluorométhoxy-phényl)-acrylamide,
(S)-N-[4-(4-Acétyl-pipérazin-1-yl)-benzyl]-N-[1-benzyl-2-(4-{4-[(2-hydroxy-éthyl)-méthyl-amino]-benzyl}-pipérazin-1-yl)-2-oxo-éthyl]-3-(4-isopropyl-phényl)-acrylamide,
(S)-N-[4-(4-Acétyl-pipérazin-1-yl)-benzyl]-N-[1-benzyl-2-(4-{4-[(2-hydroxy-éthyl)-méthyl-amino]-benzyl}-pipérazin-1-yl)-2-oxo-éthyl]-3-(4-*tert*-butyl-phényl)-acrylamide et
(S)-N-[4-(4-Acétyl-pipérazin-1-yl)-benzyl]-N-{1-benzyl-2-[4-(4-nitro-benzyl)-pipérazin-1-yl]-2-oxo-éthyl}-3-(4-trifluorométhyl-phényl)-acrylamide,
ou sels de ces composés.

17. Composé selon la revendication 1, sélectionné dans le groupe consistant en les suivants :
(S)-N-[4-(4-Acétyl-pipérazin-1-yl)benzyl]-N-{1-(4-(4-(diméthylamino)benzyl)pipérazin-1-yl)-1-oxo-3-phénylpropan-2-yl}cinnamamide,
(S)-N-[4-(4-Acétyl-pipérazin-1-yl)benzyl]-N-{1-[4-(4-(diméthylamino)benzyl)pipérazin-1-yl]-1-oxo-3-phénylpropan-2-yl}-3-(*p*-tolyl)acrylamide,
(S)-N-[4-(4-Acétyl-pipérazin-1-yl)benzyl]-N-{1-(4-(4-(diméthylamino)benzyl)pipérazin-1-yl)-1-oxo-3-phénylpropan-2-yl}-3-(4-propylphényl)acrylamide,
(S)-N-[4-(4-Acétyl-pipérazin-1-yl)benzyl]-N-{1-(4-(4-(diméthylamino)benzyl)pipérazin-1-yl)-1-oxo-3-phénylpropan-2-yl}-3-(4-méthoxyphényl)acrylamide,
(S)-3-(4-Acétyl-phényl)-N-[4-(4-acétyl-pipérazin-1-yl)benzyl]-N-{1-(4-(4-(diméthylamino)benzyl)pipérazin-1-yl)-1-oxo-3-phénylpropan-2-yl}acrylamide,
(S)-N-[4-(4-Acétyl-pipérazin-1-yl)benzyl]-3-(4-cyanophényl)-N-{1-(4-(4-(diméthylamino)benzyl)pipérazin-1-yl)-1-oxo-3-phénylpropan-2-yl}acrylamide,
(S)-N-[4-(4-Acétyl-pipérazin-1-yl)benzyl]-N-{1-(4-(4-(diméthylamino)benzyl)pipérazin-1-yl)-1-oxo-3-phénylpropan-2-yl}-3-(4-fluorophényl)acrylamide et
(S)-N-[4-(4-Acétyl-pipérazin-1-yl)benzyl]-3-(4-chlorophényl)-N-{1-(4-(4-(diméthylamino)benzyl)pipérazin-1-yl)-1-oxo-3-phénylpropan-2-yl}acrylamide,
ou sels de ces composés.

18. Composé selon la revendication 1, sélectionné dans le groupe consistant en les suivants :
(S)-N-[4-(4-Acétyl-pipérazin-1-yl)-benzyl]-N-[1-benzyl-2-(4-benzyl-pipérazin-1-yl)-2-oxo-éthyl]-3-(4-éthyl-phényl)-acrylamide,
(S)-N-[4-(4-Acétyl-pipérazin-1-yl)-benzyl]-N-[1-benzyl-2-(4-benzyl-pipérazin-1-yl)-2-oxo-éthyl]-3-(4-*tert*-butyl-phényl)-acrylamide,
(S)-N-[4-(4-Acétyl-pipérazin-1-yl)-benzyl]-N-[1-benzyl-2-(4-benzyl-pipérazin-1-yl)-2-oxo-éthyl]-3-(4-éthoxy-phényl)-acrylamide,
Ester méthylique de l'acide (S)-4-(2-{[4-(4-acétyl-pipérazin-1-yl)-benzyl]-[l-benzyl-2-(4-benzyl-pipérazin-1-yl)-2-oxo-éthyl]-carbamoyl}-vinyl)-benzoïque,
(S)-N-[4-(4-Acétyl-pipérazin-1-yl)-benzyl]-N-[1-benzyl-2-(4-benzyl-pipérazin-1-yl)-2-oxo-éthyl]-3-(4-méthanesulfonyl-phényl)-acrylamide,
(S)-3-(4-Acétylamino-phényl)-N-[4-(4-acétyl-pipérazin-1-yl)-benzyl]-N-[1-benzyl-2-(4-benzyl-pipérazin-1-yl)-2-oxo-éthyl]-acrylamide,
(S)-N-[4-(4-Acétyl-pipérazin-1-yl)-benzyl]-N-[1-benzyl-2-(4-benzyl-pipérazin-1-yl)-2-oxo-éthyl]-3-(4-propoxy-phényl)-acrylamide,
(S)-N-[4-(4-Acétyl-pipérazin-1-yl)-benzyl]-N-[1-benzyl-2-(4-benzyl-pipérazin-1-yl)-2-oxo-éthyl]-3-(4-isopropoxy-phényl)-acrylamide,
(S)-N-[4-(4-Acétyl-pipérazin-1-yl)-benzyl]-N-[1-benzyl-2-(4-benzyl-pipérazin-1-yl)-2-oxo-éthyl]-3-(4-isopropyl-phényl)-acrylamide,
(S)-N-[4-(4-Acétyl-pipérazin-1-yl)-benzyl]-N-{1-benzyl-2-[4-(4-cyano-benzyl)-pipérazin-1-yl]-2-oxo-éthyl}-3-(4-trifluorométhyl-phényl)-acrylamide,
(S)-N-[4-(4-Acétyl-pipérazin-1-yl)-benzyl]-N-{1-benzyl-2-[4-(4-cyano-benzyl)-pipérazin-1-yl]-2-oxo-éthyl}-3-(4-éthoxy-phényl)-acrylamide,
(S)-N-[4-(4-Acétyl-pipérazin-1-yl)-benzyl]-N-{1-benzyl-2-[4-(4-cyano-benzyl)-pipérazin-1-yl]-2-oxo-éthyl}-3-(4-méthanesulfonyl-phényl)-acrylamide,
(S)-3-(4-Acétylamino-phényl)-N-[4-(4-acétyl-pipérazin-1-yl)-benzyl]-N-{1-benzyl-2-[4-(4-cyano-benzyl)-pipérazin-1-yl]-2-oxo-éthyl}-acrylamide,
(S)-N-[4-(4-Acétyl-pipérazin-1-yl)-benzyl]-N-{1-benzyl-2-[4-(4-cyano-benzyl)-pipérazin-1-yl]-2-oxo-éthyl}-3-(4-propoxy-phényl)-acrylamide,
(S)-N-[4-(4-Acétyl-pipérazin-1-yl)-benzyl]-N-{1-benzyl-2-[4-(4-cyano-benzyl)-pipérazin-1-yl]-2-oxo-éthyl}-3-(4-isopropoxy-phényl)-acrylamide,
(S)-N-[4-(4-Acétyl-pipérazin-1-yl)-benzyl]-N-{1-benzyl-2-[4-(4-cyano-benzyl)-pipérazin-1-yl]-2-oxo-éthyl}-3-(4-éthyl-phényl)-acrylamide,
(S)-N-[4-(4-Acétyl-pipérazin-1-yl)-benzyl]-N-{1-benzyl-2-[4-(4-cyano-benzyl)-pipérazin-1-yl]-2-oxo-éthyl}-3-(4-difluorométhoxy-phényl)-acrylamide,
(S)-N-[4-(4-Acétyl-pipérazin-1-yl)-benzyl]-N-{1-benzyl-2-[4-(4-cyano-benzyl)-pipérazin-1-yl]-2-oxo-éthyl}-3-(4-isopropyl-phényl)-acrylamide,
(S)-N-[4-(4-Acétyl-pipérazin-1-yl)-benzyl]-N-{1-benzyl-2-[4-(4-cyano-benzyl)-pipérazin-1-yl]-2-oxo-éthyl}-3-(4-trifluorométhoxy-phényl)-acrylamide,
(S)-N-[4-(4-Acétyl-pipérazin-1-yl)-benzyl]-N-{1-benzyl-2-[4-(4-cyano-benzyl)-pipérazin-1-yl]-2-oxo-éthyl}-3-(4-*tert*-butyl-phényl)-acrylamide,
(S)-N-[4-(4-Acétyl-pipérazin-1-yl)-benzyl]-N-{1-benzyl-2-[4-(4-chloro-benzyl)-pipérazin-1-yl]-2-oxo-éthyl}-3-(4-éthoxy-phényl)-acrylamide,
(S)-N-[4-(4-Acétyl-pipérazin-1-yl)-benzyl]-N-{1-benzyl-2-[4-(4-chloro-benzyl)-pipérazin-1-yl]-2-oxo-éthyl}-3-(4-méthanesulfonyl-phényl)-acrylamide,
(S)-3-(4-Acétylamino-phényl)-N-[4-(4-acétyl-pipérazin-1-yl)-benzyl]-N-{1-benzyl-2-[4-(4-chloro-benzyl)-pipérazin-1-yl]-2-oxo-éthyl}-acrylamide,
(S)-N-[4-(4-Acétyl-pipérazin-1-yl)-benzyl]-N-{1-benzyl-2-[4-(4-chloro-benzyl)-pipérazin-1-yl]-2-oxo-éthyl}-3-(4-propoxy-phényl)-acrylamide,
(S)-N-[4-(4-Acétyl-pipérazin-1-yl)-benzyl]-N-{1-benzyl-2-[4-(4-chloro-benzyl)-pipérazin-1-yl]-2-oxo-éthyl}-3-(4-isopropoxy-phényl)-acrylamide,
(S)-N-[4-(4-Acétyl-pipérazin-1-yl)-benzyl]-N-{1-benzyl-2-[4-(4-chloro-benzyl)-pipérazin-1-yl]-2-oxo-éthyl}-3-(4-éthyl-phényl)-acrylamide,
(S)-N-[4-(4-Acétyl-pipérazin-1-yl)-benzyl]-N-{1-benzyl-2-[4-(4-chloro-benzyl)-pipérazin-1-yl]-2-oxo-éthyl}-3-(4-difluorométhoxy-phényl)-acrylamide,
(S)-N-[4-(4-Acétyl-pipérazin-1-yl)-benzyl]-N-{1-benzyl-2-[4-(4-chloro-benzyl)-pipérazin-1-yl]-2-oxo-éthyl}-3-(4-isopropyl-phényl)-acrylamide,
(S)-N-[4-(4-Acétyl-pipérazin-1-yl)-benzyl]-N-{1-benzyl-2-[4-(4-chloro-benzyl)-pipérazin-1-yl]-2-oxo-éthyl}-3-(4-*tert*-butyl-phényl)-acrylamide,
(S)-N-[4-(4-Acétyl-pipérazin-1-yl)-benzyl]-N-{1-benzyl-2-[4-(4-méthoxy-benzyl)-pipérazin-1-yl]-2-oxo-éthyl}-3-(4-trifluorométhyl-phényl)-acrylamide et
(S)-N-[4-(4-Acétyl-pipérazin-1-yl)-benzyl]-N-{1-benzyl-2-[4-(4-isopropoxy-benzyl)-pipérazin-1-yl]-2-oxo-éthyl}-3-(4-trifluorométhyl-phényl)-acrylamide,
ou sels de ces composés.

19. Composé selon la revendication 1, sélectionné dans le groupe consistant en les suivants :
(S)-N-[4-(4-Acétyl-pipérazin-1-yl)-benzyl]-N-{1-benzyl-2-[4-(4-(méthylsulfonyl)-benzyl)-pipérazin-1-yl]-2-oxo-éthyl}-3-(4-isopropoxyphényl)-acrylamide,
(S)-N-[4-(4-Acétyl-pipérazin-1-yl)-benzyl]-N-{1-benzyl-2-[4-(4-(méthylsulfonyl)-benzyl)-pipérazin-1-yl]-2-oxo-éthyl}-3-(4-*tert*-butyl-phényl)-acrylamide,
(S)-N-[4-(4-Acétyl-pipérazin-1-yl)-benzyl]-N-{1-benzyl-2-[4-(4-(méthylsulfonyl)-benzyl)-pipérazin-1-yl]-2-oxo-éthyl}-3-(4-propoxyphényl)-acrylamide,
(S)-N-(4-(4-Acétylpipérazin-1-yl)benzyl)-N-(1-(4-(4-(méthylsulfonyl)benzyl)pipérazin-1-yl)-1-oxo-3-phénylpropan-2-yl)-3-(4-(trifluorométhyl)phényl)acrylamide,
(S)-N-[4-(4-Acétyl-pipérazin-1-yl)-benzyl]-N-{1-benzyl-2-[4-(4-(éthylsulfonyl)-benzyl)-pipérazin-1-yl]-2-oxo-éthyl}-3-(4-propoxyphényl)-acrylamide et
(S)-N-(4-(4-Acétylpipérazin-1-yl)benzyl)-N-(1-(4-(4-(éthylsulfonyl)benzyl)pipérazin-1-yl)-1-oxo-3-phénylpropan-2-yl)-3-(4-(trifluorométhyl)phényl)acrylamide,
ou sels de ces composés.

20. Composition pharmaceutique comprenant un composé selon l'une quelconque des revendications 1 à 19 ou un sel pharmaceutiquement acceptable de celui-ci et un véhicule pharmaceutiquement acceptable.

21. Composé selon l'une quelconque des revendications 1 à 19 ou sel pharmaceutiquement acceptable de celui-ci, ou composition selon la revendication 20, en vue d'une utilisation en tant que médicament.

22. Composé selon l'une quelconque des revendications 1 à 19 ou sel pharmaceutiquement acceptable de celui-ci, pour une utilisation dans le traitement et/ou la prévention d'infections à protozoaires.

23. Composé selon l'une quelconque des revendications 1 à 19 ou sel pharmaceutiquement acceptable de celui-ci, pour une utilisation dans le traitement et/ou la prévention du paludisme.

24. Utilisation d'un composé selon l'une quelconque des revendications 1 à 19 ou d'un sel pharmaceutiquement acceptable de celui-ci dans la préparation d'une composition pharmaceutique indiquée dans le traitement et/ou la prévention d'infections à protozoaires.

25. Utilisation selon la revendication 24 dans le traitement et/ou la prévention du paludisme.
